(19) Europäisches Patentamt
European Patent Office
Office européen des brevets

(11) Publication number: 0 423 777 A2

# (12) EUROPEAN PATENT APPLICATION

(21) Application number: 90119945.5

(22) Date of filing: 18.10.90

(51) Int. Cl.5: C07H 19/167, A61K 31/70

(30) Priority: 19.10.89 US 423913

(43) Date of publication of application:
24.04.91 Bulletin 91/17

(84) Designated Contracting States:
AT BE CH DE DK ES FR GB GR IT LI LU NL SE

(71) Applicant: G.D. Searle & Co.
P.O. Box 5110
Chicago Illinois 60680(US)

(72) Inventor: Becker, Daniel Paul
1427 Pebble Creek Drive
USA-Glenview, Illinois 60025(US)

Inventor: Collins, Paul Waddell
1557 Hawthorne Place
USA-Deerfield, Illinois 60015(US)
Inventor: Flynn, Daniel Lee
17 North Bristol Ct.
USA-Mundelein, Illinois 60060(US)
Inventor: Gullikson, Gary William
201 Court of Ash
USA-Vernon Hills, Illinois 60061(US)

(74) Representative: Beil, Hans Chr., Dr. et al
Beil, Wolff und Beil, Rechtsanwälte
Adelonstrasse 58 Postfach 80 01 40
W-6230 Frankfurt am Main 80(DE)

## (54) Method of treating gastrointestinal motility disorders.

(57) The present invention relates to a method of treating gastrointestinal motility disorders of a mammal by administering to the mammal in need thereof a therapeutically effective amount of a N(6) substituted adenosine compound of the general formula

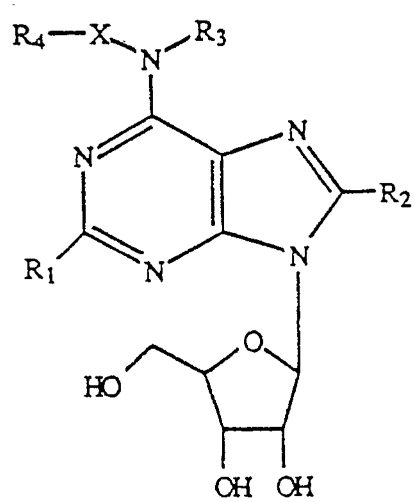

or a pharmaceutically acceptable salt thereof.

EP 0 423 777 A2

# METHOD OF TREATING GASTROINTESTINAL MOTILITY DISORDERS

## Background of the Invention

The invention herein is directed to a method of treating gastrointestinal motility disorders of a mammal by administering to the mammal in need thereof a therapeutically effective amount of a compound disclosed herein or a pharmaceutically acceptable salt thereof. The compounds used herein are purine and adenosine derived compounds. The method can be practiced to treat gastrointestinal motility disorders such as gastroesophageal reflux, diseases characterized by delayed gastric emptying, ileus, irritable bowel syndrome, and the like. The compounds disclosed herein have been found to exhibit gastrointestinal prokinetic activity and are therefore, useful in treating gastrointestinal motility disorders.

Some of the compounds disclosed as being gastrointestinal prokinetic agents herein have heretofore been found to be useful as cardiovascular/coronary vasodilators, platelet aggregation inhibitors, growth reGulators and anti-neoplastic agents. Some of the compounds have heretofore demonstrated biological activity, such as smooth muscle contractility and adenylate cyclase activity, anti-hypertensive activity, anti-allergic activity, anti-lipolytic activity and anti-hyperlipaemic activity.

Japanese patent 49/30396 of Kohjin company Limited; Japanese patent 49/30395, also of Kohjin Company Limited; U. S. Patent 3,901,876 of Schering; U. S. Patent 3,551,409 of Boehringer Mannheim; U. S. Patent 4,464, 361 of Fujisawa Pharmaceutical Company Limited; and West German Patent 2139107 of Merck Patent GmbH all disclose utility for various adenosine derivatives as cardiovascular agents or coronary vasodilators, anti-hypertensive agents, bradycardiac agents or central nervous system agents.

U. S. Patent 4,340,730 of G. D. Searle & Co. discloses anti-hypertensive activity for certain adenosine derivatives.

K. Kikugawa, et al. disclose in J. Med. Chem. 16 , 358 (1973) adenosine derivatives which act as platelet aggregation inhibitors.

S. P. Dutta, et al. disclose in J. Carbohydrates, Nucleosides, Nucleotides , 5 , 47 (1978) that certain adenosine derivatives have utility as growth regulators, anti-neoplastic agents and exhibit cytokinin activity.

H. P. Baer has found that certain adenosine derivatives exhibit smooth muscle contractility and adenylate cyclase activity in in vitro studies, Can. J. of Physiology Pharmacol. 63 , 58 (1985).

U. S. Patent 4,704,381 of Boehringer Mannheim discloses certain adenosine derivatives that exhibit anti-allergic activity and which, therefore, can be used as anti-allergic agents.

European patent 0061001A1 of Yamasa Shoyu discloses that certain adenosine derivatives exhibit anti-allergic activity.

U. S. Patent 3,851,056 of Boehringer Mannheim discloses certain adenosine derivatives which exhibit anti-lipolytic and anti-hyperlipaemic activity.

## Summary of the Invention

The invention herein is directed to a method of treating gastrointestinal motility disorders by administering to a mammal in need of such a treatment a therapeutically effective amount of a compound of the formula

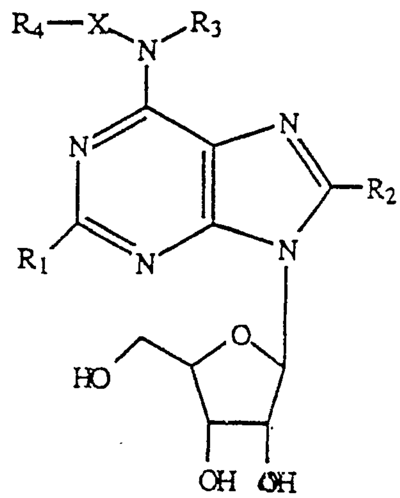

wherein each $R_1$ and $R_2$ is independently hydrogen, hydroxyl, halogen, alkyl, phenyl, alkoxy, morpholino, piperidino, piperazino, phenoxy, thiophenoxy or amino optionally substituted by alkyl, aralkyl, or phenyl;
wherein each $R_3$ can independently be hydrogen or lower alkyl;
wherein X can be a straight chain, branched chain or cyclic alkylene from 1 to 10 carbon atoms; and wherein $R_4$ can be optionally substituted imidazol-1-yl, imidazol-2-yl, imidazol 4-yl, pyrrolinyl, pyrrolidinyl, piperidinyl, triazolyl, hydroxyalkyl, dihydroxyalkyl, alkoxy alkyl, and dialkoxyalkyl,

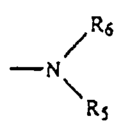

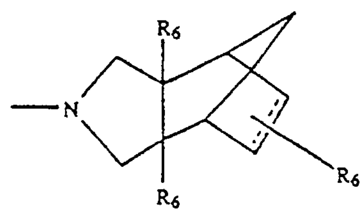

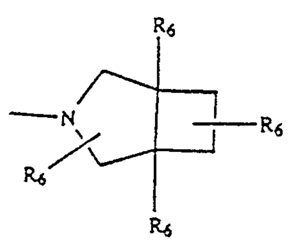

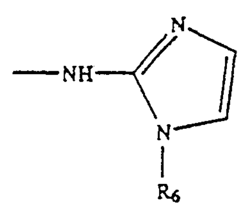

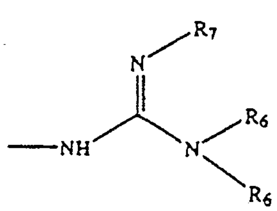

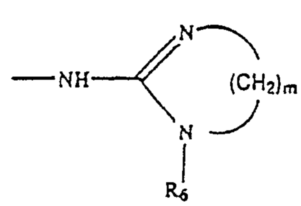

wherein X can be a valence bond and $R_4$ can be

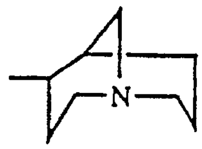

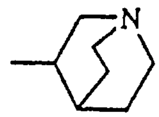

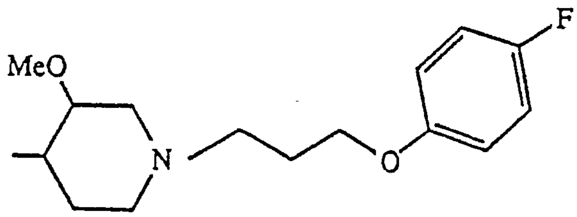

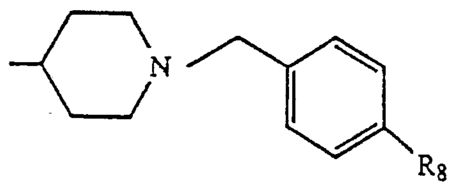

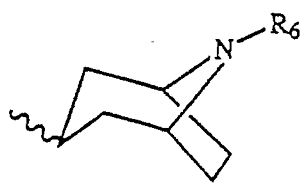

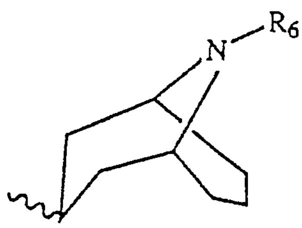

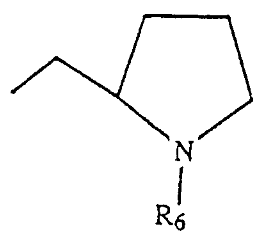

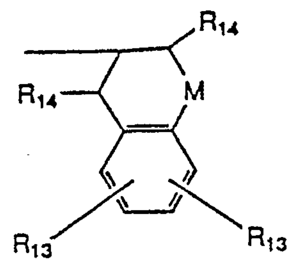

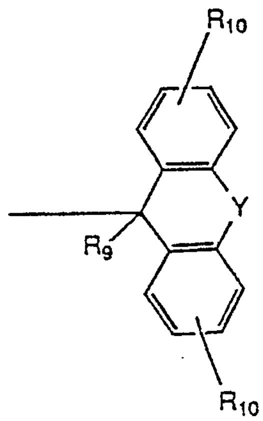

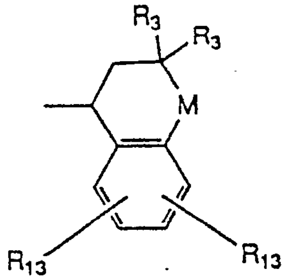

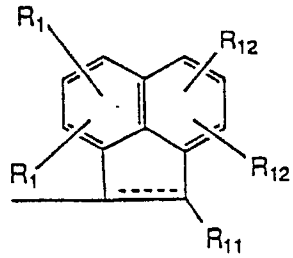

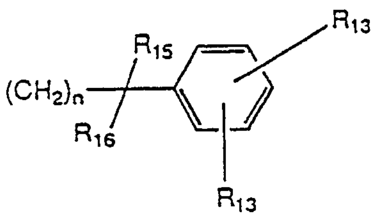

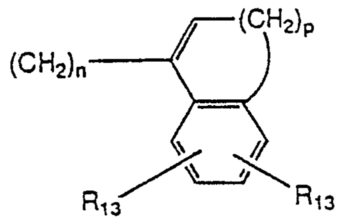

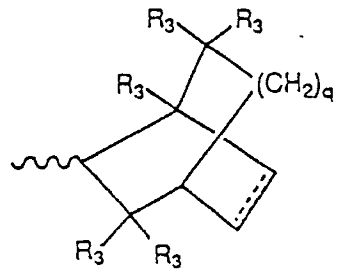

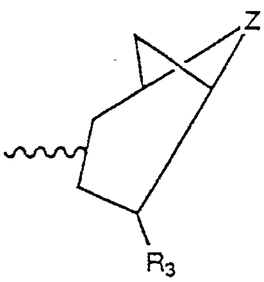

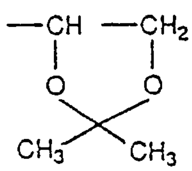

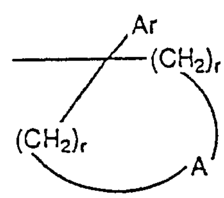

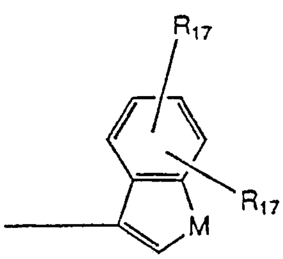

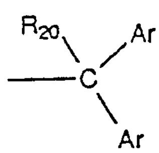

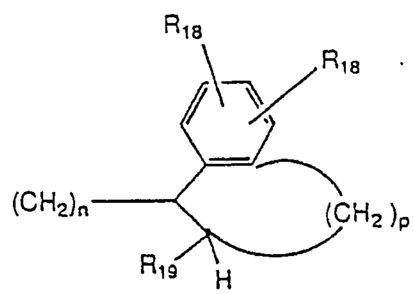

wherein each $R_5$ and $R_6$ can independently be hydrogen, alkyl, aralkyl, phenyl, and optionally substituted phenyl and aralkyl; and

wherein each $R_7$ can independently be hydrogen, alkyl, aralkyl, aryl, cyano or nitro;

wherein each $R_8$ can independently be hydrogen, alkyl, aralkyl, aryl or acyl;

wherein each $R_9$ can independently be hydrogen, hydroxy, lower alkyl, lower alkanoyloxy and benzoyloxy;

wherein each $R_{10}$ can independently be hydrogen, halogen, hydroxy, lower alkoxy, lower alkanoyloxy, benzoyloxy, lower $S(O)_q$ - alkyl, sulfonamide, trifluoromethyl, lower alkyl, amino, lower mono- or dialkylamino, nitro or sulfhydryl;

wherein each $R_{11}$ can independently be hydrogen, lower alkyl, hydroxy or lower alkoxy;

wherein each $R_{12}$ can independently be hydrogen, hydroxy, halogen, amino, nitro, lower alkyl, lower alkoxy or trifluoromethyl;

wherein each $R_{13}$ can independently be hydrogen, lower alkyl, lower alkoxy, hydroxy, lower alkanoyl, nitro, amino, trifluormethyl, halogen or taken together a methylenedioxy group;

wherein each $R_{14}$ can independently be hydrogen, hydroxy, or lower alkyl;

wherein each $R_{15}$ can independently be a straight or branched lower alkyl;

wherein each $R_{16}$ can independently be hydrogen, lower alkyl, lower alkoxy, or lower alkanoyloxy;

wherein each $R_{17}$ can independently be hydrogen, hydroxy, lower alkoxy, lower alkyl, amino, monoloweralkyl amino, diloweralkyl amino, nitro or halogen;

wherein each $R_{18}$ can independently be hydrogen, lower alkyl, lower alkoxy, hydroxY, lower alkanoyl, nitro, trifluoromethyl, halogen, amino, monoloweralkyl amino, diloweralkyl amino, or taken together are a methylenedioxy group;

wherein each $R_{19}$ can independently be hydrogen, lower alkyl or lower alkanoyl;

wherein each $R_{20}$ can independently be hydrogen, hydroxy, lower alkyl, lower carboalkoxy, or lower alkanoyloxy;

wherein Y can be a valence bond, O, $S(O)_q$, NR in which R is H, lower alkyl, lower alkanoyl or benzoyl, $(CH_2)_s$, or -CH=CH-

wherein M can be O, S, SO or $SO_2$;

wherein Z can be $-C(CH_3)_2-$, $-CH_2-$, $-CH_2-CH_2-$ or -CH=CH-;

wherein A can be a valence bond, O, S,

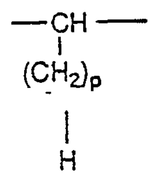

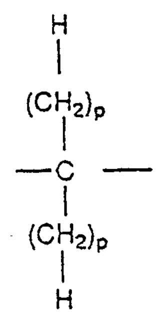

wherein each Ar can independently be (1) phenyl, (2) 1- or 2-naphthalenyl, (3)2-or 3-thienyl, (4)2-or 3-furanyl, (5)2-,4-, or 5-thiazyl (6) 2-, 3-, or 4-pyridyl, or (7)2-pyrimidyl wherein each of (1), (2), (3), (4), (5), (6) or (7) is unsubstituted or substituted with at least one of lower alkyl, halogen, trifluoromethyl, hydroxy, lower alkoxy, lower acyloxy, amino, N-lower monoalkyl or N,N-lower dialkylamino, lower thioalkyl, lower alkylsulfonyl, or nitro;

wherein m can be 2 or 3;

wherein n can be an an integer from 0 to 4;

wherein p can be an integer from 1 to 4;

wherein q can be an integer from 0 to 2;

wherein r can be an integer from 0 to 3;

wherein s can be 1 or 2; or

a pharmaceutically acceptable salt thereof.

## Detailed Description of the Invention

The invention herein is a method of treating gastrointestinal motility disorders to a mammal in need of such treatment by administering to such mammal a therapeutically effective amount of a compound of the formula:

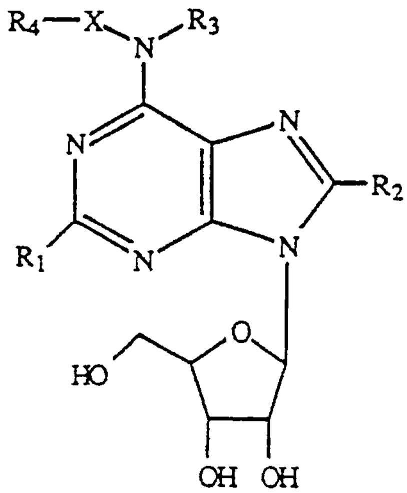

wherein each $R_1$ and $R_2$ can be independently hydrogen, hydroxyl, halogen, alkyl, phenyl, alkoxy, morpholino, piperidino, piperazino, phenoxy, thiophenoxy or amino optionally substituted by alkyl, aralkyl, or phenyl;

wherein each $R_3$ can independently be hydrogen or lower alkyl;

wherein X can be a straight chain, branched chain or cyclic alkylene from 1 to 10 carbon atoms; and wherein $R_4$ can be optionally substituted imidazol-1-yl, imidazol-2-yl, imidazol-4-yl, pyrrolinyl, pyrrolidinyl, piperidinyl, triazolyl, hydroxyalkyl, dihydroxyalkyl, alkoxy alkyl, and dialkoxyalkyl,

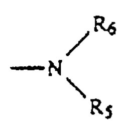

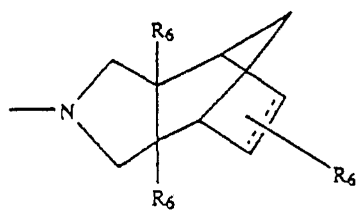

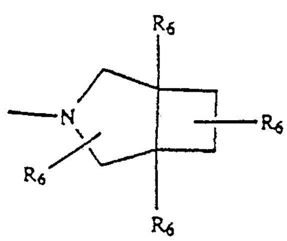

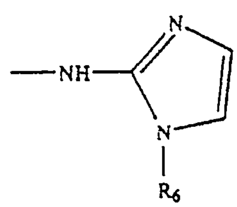

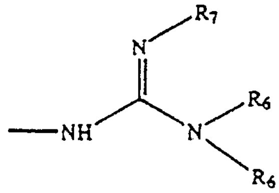

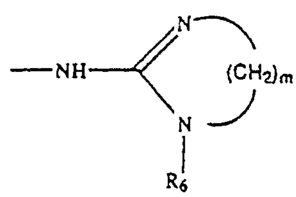

wherein X can be a valence bond and $R_4$ can be

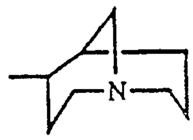

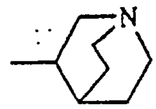

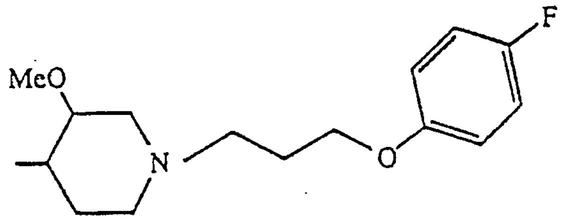

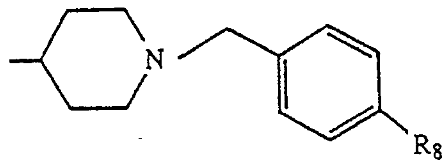

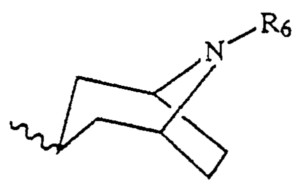

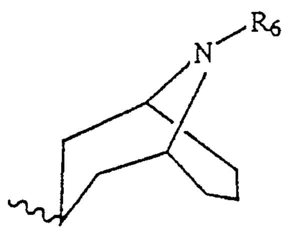

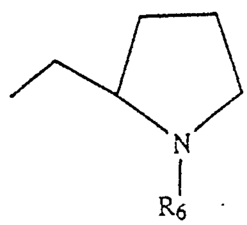

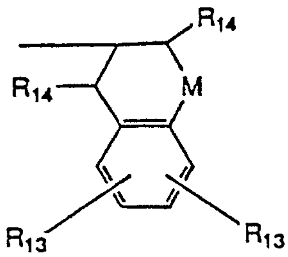

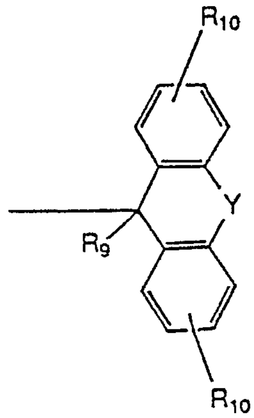

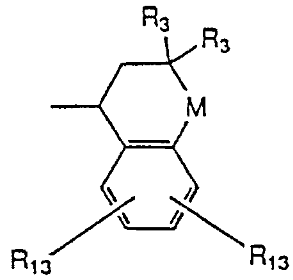

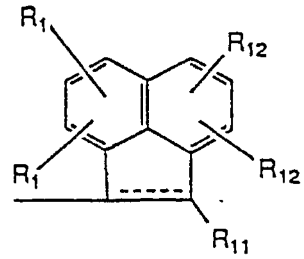

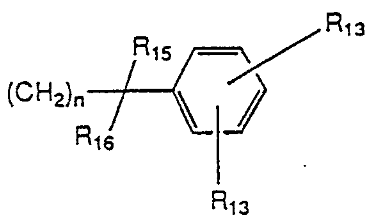

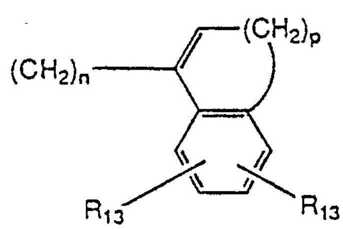

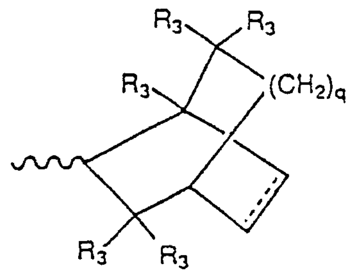

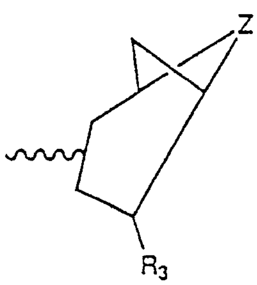

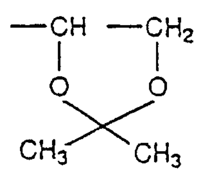

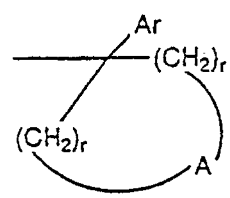

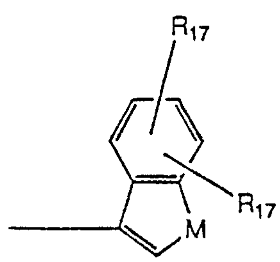

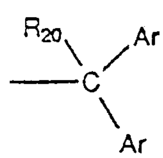

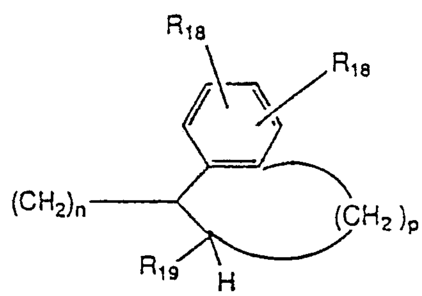

wherein each $R_5$ and $R_6$ can independently be hydrogen, alkyl, aralkyl, phenyl, and optionally substituted phenyl and aralkyl; and

wherein each $R_7$ can independently be hydrogen, alkyl, aralkyl, aryl, cyano or nitro;

wherein each $R_8$ can independently be hydrogen, alkyl, aralkyl, aryl or acyl;

wherein each $R_9$ can independently be hydrogen, hydroxy, lower alkyl, lower alkanoyloxy and benzoyloxy;

wherein each $R_{10}$ can independently be hydrogen, halogen, hydroxy, lower alkoxy, lower alkanoyloxy, benzoyloxy, lower $S(O)_q$ - alkyl, sulfonamide, trifluoromethyl, lower alkyl, amino, lower mono- or dialkylamino, nitro or sulfhydryl;

wherein each $R_{11}$ can independently be hydrogen, lower alkyl, hydroxy or lower alkoxy;
wherein each $R_{12}$ can independently be hydrogen, hydroxy, halogen, amino, nitro, lower alkyl, lower alkoxy or trifluoromethyl;
wherein each $R_{13}$ can independently be hydrogen, lower alkyl, lower alkoxy, hydroxy, lower alkanoyl, nitro, amino, trifluormethyl, halogen or taken together a methylenedioxy group;
wherein each $R_{14}$ can independently be hydrogen, hydroxy, or lower alkyl;
wherein each $R_{15}$ can independently be a straight or branched lower alkyl;
wherein each $R_{16}$ can independently be hydrogen, lower alkyl, lower alkoxy, or lower alkanoyloxy;
wherein each $R_{17}$ can independently be hydrogen, hydroxy, lower alkoxy, lower alkyl, amino, monoloweralkyl amino, diloweralkyl amino, nitro or halogen;
wherein each $R_{18}$ can independently be hydrogen, lower alkyl, lower alkoxy, hydroxy, lower alkanoyl, nitro, trifluoromethyl, halogen, amino, monoloweralkyl amino, diloweralkyl amino, or taken together are a methylenedioxy group;
wherein each $R_{19}$ can independently be hydrogen, lower alkyl or lower alkanoyl;
wherein each $R_{20}$ can independently be hydrogen, hydroxy, lower alkyl, lower carboalkoxy, or lower alkanoyloxy;
wherein Y can be a valence bond, O, $S(O)_q$, NR in which R is H, lower alkyl, lower alkanoyl or benzoyl, $(CH_2)_s$, or -CH=CH-
wherein M can be O, S, SO or $SO_2$;
wherein Z can be $-C(CH_3)_2-$, $-CH_2-$, $-CH_2-CH_2-$ or -CH=CH -
wherein A can be a valence bond, O, S,

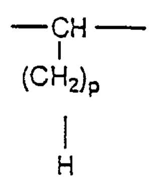
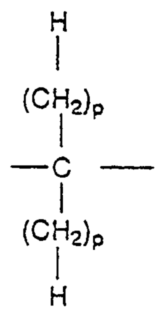

wherein each Ar can independently be (1) phenyl, (2) 1- or 2-naphthalenyl, (3)2-or 3-thienyl, (4)2-or 3-furanyl, (5)2-,4-, or 5-thiazyl (6) 2-, 3-, or 4-pyridyl, or (7)2-pyrimidyl wherein each of (1), (2), (3), (4), (5), (6) or (7) is unsubstituted or substituted with at least one of lower alkyl, halogen, trifluoromethyl, hydroxy, lower alkoxy, lower acyloxy, amino, N-lower monoalkyl or N,N-lower dialkylamino, lower thioalkyl, lower alkylsulfonyl, or nitro;
wherein m can be 2 or 3;
wherein n can be an integer from 0 to 4;
wherein p can be an integer from 1 to 4;
wherein q can be an integer from 0 to 2;
wherein r can be an integer from 0 to 3;
wherein s can be 1 or 2; or
a pharmaceutically acceptable salt thereof.

A preferred method of treating gastrointestinal motility disorders is performed by administering to a mammal in need of such treatment a therapeutically effective amount of a compound of the formula:

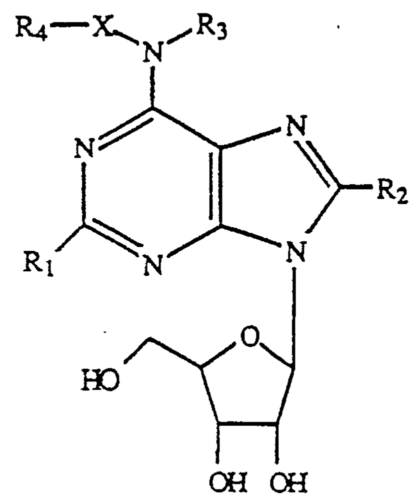

wherein each $R_1$ and $R_2$ is independently hydrogen, hydroxyl, halogen, alkyl, phenyl, alkoxy, morpholino, piperidino, piperazino, phenoxy, thiophenoxy or amino optionally substituted by alkyl, aralkyl, or phenyl;
wherein each $R_3$ can independently be hydrogen or lower alkyl;
wherein X can be a straight chain, branched chain or cyclic alkylene from 1 to 10 carbon atoms; and wherein $R_4$ can be optionally substituted imidazol-1-yl, imidazol-2-yl, imidazol-4-yl, pyrrolinyl, pyrrolidinyl, piperidinyl, triazolyl, hydroxyalkyl, dihydroxyalkyl, alkoxy alkyl, dialkoxyalkyl,

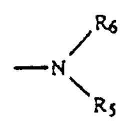

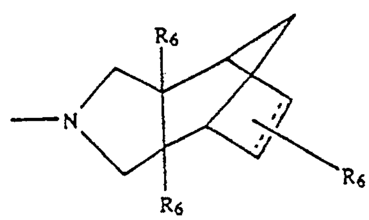

:

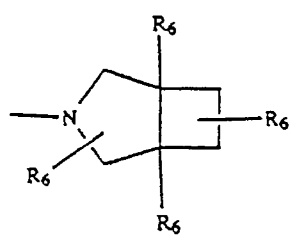

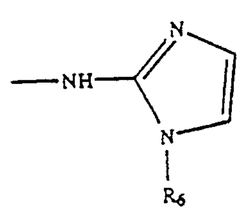

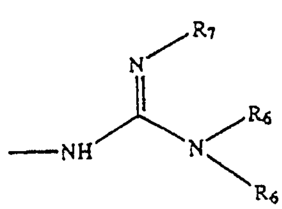

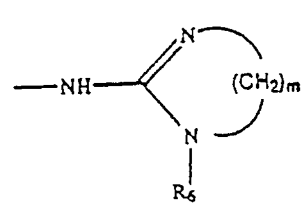

or a pharmaceutically acceptable salt thereof and wherein $R_5$, $R_6$, $R_7$ and m are as defined above.

Another preferred method of treating gastrointestinal motility disorders is performed by administering to a mammal in need of such treatment a therapeutically effective amount of a compound of the formula:

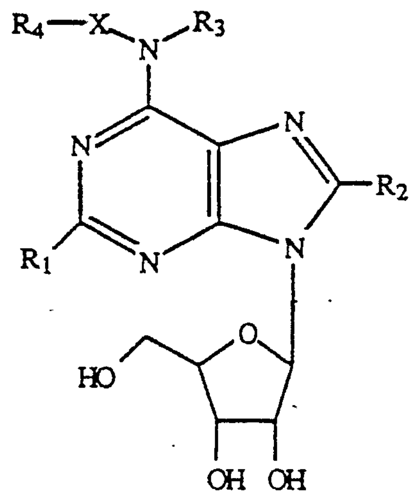

wherein each $R_1$ and $R_2$ is independently hydrogen, hydroxyl, halogen, alkyl, phenyl, alkoxy, morpholino, piperidino, piperazino, phenoxy, thiophenoxy or amino optionally substituted by alkyl, aralkyl, or phenyl; wherein each $R_3$ can independently be hydrogen or lower alkyl;
wherein X can be a valence bond and $R_4$ can be

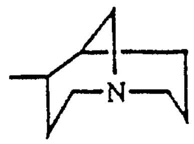

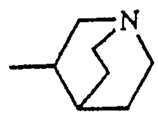

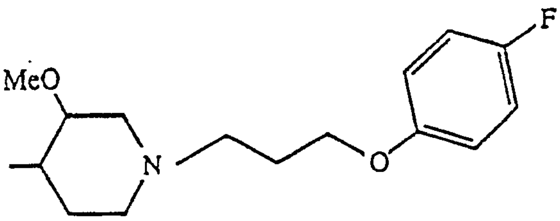

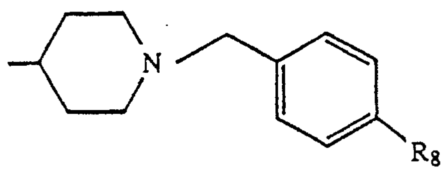

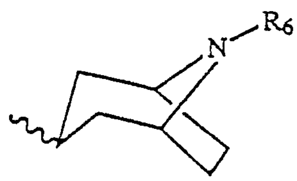

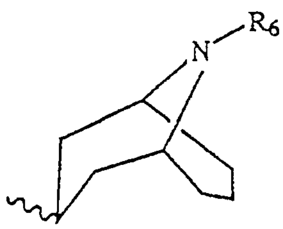

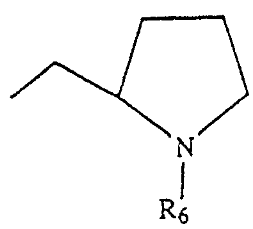

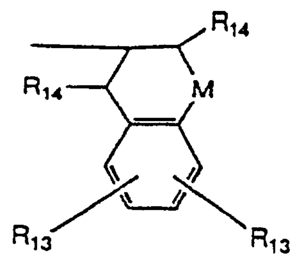

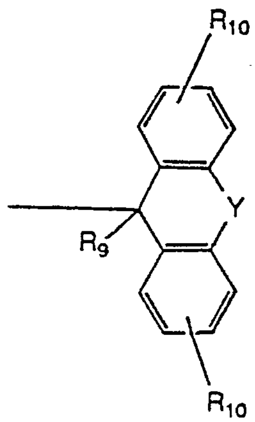

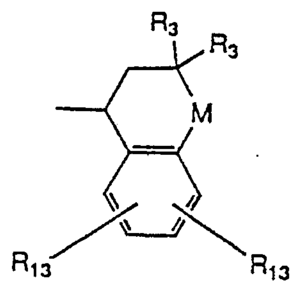

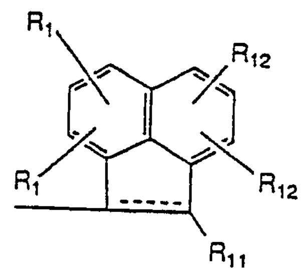

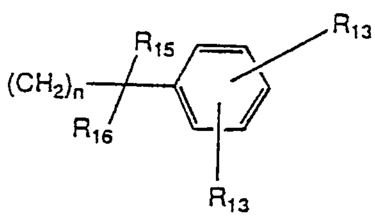

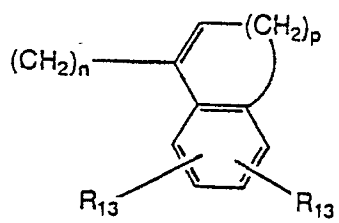

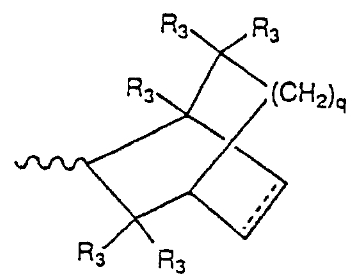

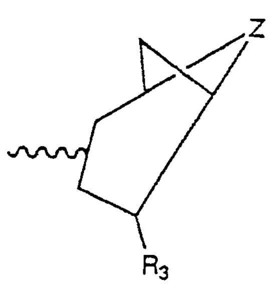

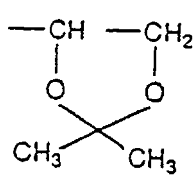

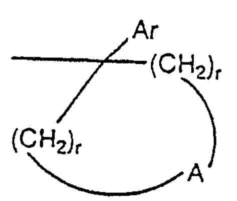

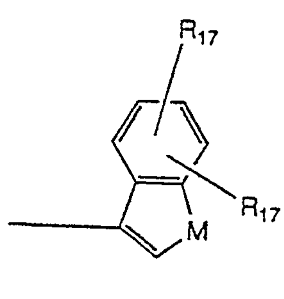

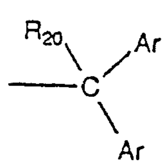

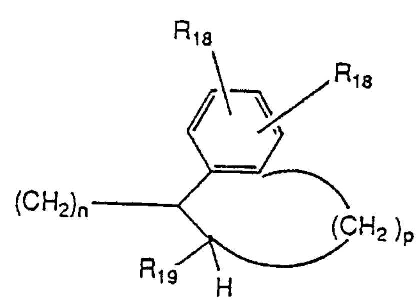

wherein each $R_6$ can independently be hydrogen, alkyl, aralkyl, phenyl, and optionally substituted phenyl and aralkyl;

wherein each $R_8$ can independently be hydrogen, alkyl, aralkyl, aryl or acyl;

wherein each $R_9$ can independently be hydrogen, hydroxy, lower alkyl, lower alkanoyloxy and benzoyloxy;

wherein each $R_{10}$ can independently be hydrogen, halogen, hydroxy, lower alkoxy, lower alkanoyloxy, benzoyloxy, lower $S(O)_Q$ - alkyl, sulfonamide, trifluoromethyl, lower alkyl, amino, lower mono- or dialkylamino, nitro or sulfhydryl;

wherein each $R_{11}$ can independently be hydrogen, lower alkyl, hydroxy or lower alkoxy;
wherein each $R_{12}$ can independently be hydrogen, hydroxy, halogen, amino, nitro, lower alkyl, lower alkoxy or trifluoromethyl;
wherein each $R_{13}$ can independently be hydrogen, lower alkyl, lower alkoxy, hydroxy lower alkanoyl, nitro, amino, trifluormethyl, halogen or taken together a methylenedioxy group;
wherein each $R_{14}$ can independently be hydrogen, hydroxy, or lower alkyl;
wherein each $R_{15}$ can independently be a straight or branched lower alkyl;
wherein each $R_{16}$ can independently be hydrogen, lower alkyl, lower alkoxy, or lower alkanoyloxy;
wherein each $R_{17}$ can independently be hydrogen, hydroxy, lower alkoxy, lower alkyl, amino, monoloweralkyl amino, diloweralkyl amino, nitro or halogen;
wherein each $R_{18}$ can independently be hydrogen, lower alkyl, lower alkoxy, hydroxy, lower alkanoyl, nitro, trifluoromethyl, halogen, amino, monoloweralkyl amino, diloweralkyl amino, or taken together are a methylenedioxy group;
wherein each $R_{19}$ can independently be hydrogen, lower alkyl or lower alkanoyl;
wherein each $R_{20}$ can independently be hydrogen, hydroxy, lower alkyl, lower carboalkoxy, or lower alkanoyloxy;
wherein Y can be a valence bond, O, $S(O)_q$, NR in which R is H, lower alkyl, lower alkanoyl or benzoyl, $(CH_2)_s$, or -CH=CH-
wherein M can be O, S, SO or $SO_2$;
wherein Z can be $-C(CH_3)_2-$, $-CH_2-$, $-CH_2-CH_2-$ or -CH=CH-
wherein A can be a valence bond, O, S,

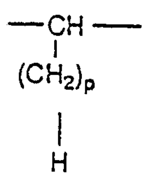

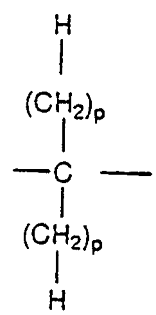

wherein each Ar can independently be (1) phenyl, (2) 1- or 2-naphthalenyl, (3)2-or 3-thienyl, (4)2-or 3-furanyl, (5)2-,4-, or 5-thiazyl (6) 2-, 3 , or 4-pyridyl, or (7)2-pyrimidyl wherein each of (1), (2), (3), (4), (5), (6) or (7) is unsubstituted or substituted with at least one of lower alkyl, halogen, trifluoromethyl, hydroxy, lower alkoxy, lower acyloxy, amino, N-lower monoalkyl or N,N-lower dialkylamino, lower thioalkyl, lower alkylsulfonyl, or nitro;
wherein n can be an integer from 0 to 4;
wherein p can be an integer from 1 to 4;
wherein q can be an integer from 0 to 2;
wherein r can be an integer from 0 to 3;
wherein s can be 1 or 2; or
a pharmaceutically acceptable salt thereof.

Another preferred method of treating gastrointestinal motility disorders in a mammal in need of such treatment includes administering a therapeutically effective amount of a compound of the formula:

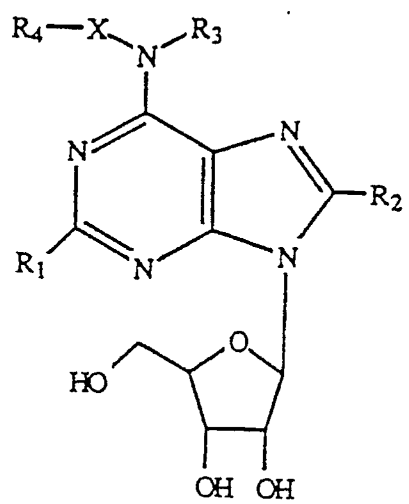

wherein $R_3$ is hydrogen and $-X-R_4$ can be:

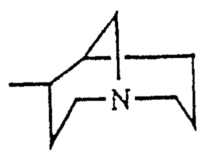

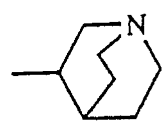

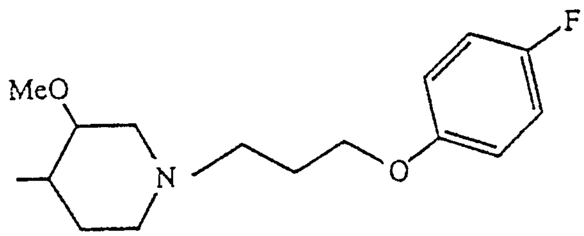

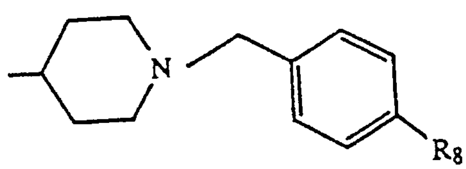

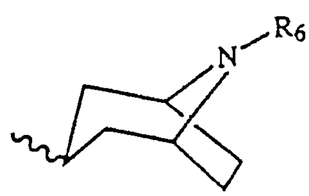

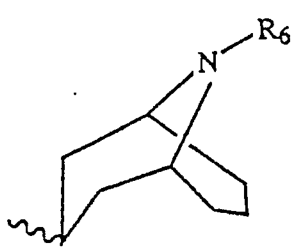

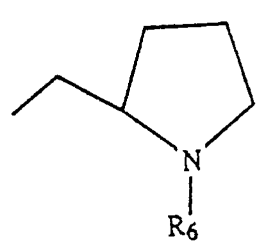

wherein each $R_1$ and $R_2$ is independently hydrogen, hydroxyl, halogen, alkyl, phenyl, alkoxy, morpholino, piperidino, piperazino, phenoxy, thiophenoxy or amino optionally substituted by alkyl, aralkyl, or phenyl;
wherein each $R_6$ can independently be hydrogen, alkyl, aralkyl, phenyl, and optionally substituted phenyl and aralkyl; and
wherein each $R_8$ can independently be hydrogen, alkyl, aralkyl, aryl or acyl; or
a pharmaceutically acceptable salt thereof.

In the structures or formulas herein the solid triangular bond representation represents a bond extending outwardly from the plane of the paper on which it is drawn. In a similar manner, the series of dashes of decreasing length are used to represent a bond extending below the plane of the paper on which the structure is drawn. A bond drawn as a wavy line represents either configuration can be present. A bond drawn generally perpendicular to the middle of a bond represents that the bond can be to either adjacent carbon atom.

In the structural formulas, a dashed line represents an optional bond between the two atoms. For example, the solid and dashed lines (a straight line and above it a series of dashes of the same length) indicate that the bond can be either a single bond or double bond between the two atoms.

The compounds that have utility in the methods described herein are not limited to any particular stereochemical configuration at the N-(6)-side chain. Both cis- and trans-isomers, where possible, are within the scope of the invention and can be used in the method described herein. In addition, geometric isomers, diastereomers, and enantiomers are also within the scope of the method herein.

The term "lower alkyl" as used herein means straight or branched chain alkyls having from 1 to 6 carbon atoms.

The term "aryl" as used herein describes phenyl or substituted phenyl.

For the purpose herein the term "thiophenoxy" is used to mean the group having the structure:

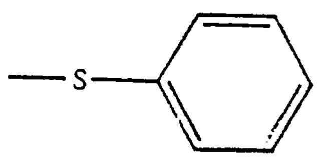

The term "pharmaceutically acceptable salts" includes acid addition salts with conventional acids including mineral acids such as hydrochloric, hydrobromic, phosphoric, sulfuric and organic acids such as ethanesulfonic, benzenesulfonic, p-toluensulfonic, citric, succinic, tartaric, lactic, acetic acid and the like. (see for example, "Pharmaceutical Salts," J. Pharm . Sci (1977)66 (1):1-19.) The pharmaceutically acceptable salts of the compounds also include quaternary ammonium salts. Examples of such salts include salts with compounds such as $R_3$-Y wherein $R_3$ is $C_{1-6}$ alkyl, phenyl-$C_{1-6}$ alkyl, or $C_{5-7}$ cycloalkyl, and Y is an anion of an acid. Suitable examples of $R_3$ include methyl, ethyl and n- and iso-propyl; and benzyl and phenyl ethyl. Suitable examples of Y include the halides such as chloride, bromide and iodide.

The acid addition salts can be prepared either by dissolving the free base in aqueous or aqueous alcohol solution or other suitable solvent containing the appropriate acid and isolating the salt by evaporating the solvent, or by reacting the free base and acid in an organic solvent, in which case the salt separates directly or can be obtained by concentration of the solution.

Quaternary ammonium salts can be prepared by reaction of the free base with an appropriate organohalide as described above. The reaction can be carried out in a solvent, such as acetone, methanol, ethanol or dimethylformamide at ambient or elevated temperature with or without pressure.

N-oxides of the compound can be formed conventionally. N-oxides of the nitrogen atom of the cyclic ring system are produced by reaction of a compound with an organic peracid such as m-chloroperbenzoic acid in, for example, a chlorinated hydrocarbon solvent at below ambient temperature.

The method disclosed herein can be used in the treatment of mammals exhibiting gastrointestinal disorders such as gastroesophageal reflux, diseases characterized by delayed gastric emptying, ileus, irritable bowel syndrome, and the like.

The method herein is practiced by administering one of the noted compounds to a mammal in need of such a treatment in a therapeutically effective amount. The compounds can be administered in such oral dosage forms as tablets, capsules, soft gels, pills, powders, granules, elixirs, or syrups. The compounds can be administered intravascularly, intraperitoneally, subcutaneously, intramuscularly, or topically, using forms known to the pharmaceutical art. In general, the preferred form of administration is oral or in such a manner so as to localize the prokinetic agent to the gastrointestinal tract. For example, it is possible to administer

the compounds via suppository.

For the oral administration of the compounds in the practice of the method herein, the foregoing compounds are administered in admixture with suitable pharmaceutical diluents, excipients, or carriers (collectively referred to herein as "carriers") suitably selected with respect to the intended form of administration, that is, oral tablets, capsules, soft gels, elixirs, syrups, drops, and the like, and consistent with conventional pharmaceutical practices.

For example, for oral administration in the form of tablets or capsules, the active drug components can be combined with any oral non-toxic pharmaceutically acceptable inert carrier, such as lactose, staroh, sucrose, cellulose, magnesium stearate, dicalcium phosphate, calcium sulfate, mannitol, and the like, or various combinations thereof. For oral administration in liquid forms, such as in soft gels, elixirs, syrups, drops, and the like, the active drug components can be combined with any oral, non-toxic pharmaceutically acceptable inert carrier, such as water, saline, ethanol, polyethylene glycol, propylene glycol, corn oil, cottonseed oil, peanut oil, sesame oil, benzyl alcohol, various buffers, and the like, or various combinations thereof. Moreover, when desired or necessary, suitable binders, lubricants, disintegrating agents, and coloring agents can also be incorporated in the mixture. Suitable binders include starch, gelatin natural sugars, corn sweeteners, natural and synthetic gums such as acacia, sodium alginate, carboxymethylcellulose, carboxyethylcellulose, polyethylene glycol, and waxes, or combinations thereof. Lubricants for use in these dosage forms include boric acid, sodium benzoate, sodium acetate, sodium chloride, and the like, or combinations thereof. Disintegrating agents include, without limitation, starch, methylcellulose, agar, bentonite, guar gum, and the like, or combinations thereof. Sweetening and flavoring agents and preservatives can also be included where appropriate.

For intravascular, intraperitoneal, subcutaneous, intramuscular, suppository or aerosol administration, active drug components can be combined with a suitable carrier such as water, saline, aqueous dextrose, and the like. Regardless of the route of administration selected, the compounds described as useful in the method herein can be formulated into pharmaceutically acceptable dosage forms by conventional methods known to those skilled in the art. The compounds can be formulated using pharmacologically acceptable acid addition salts. Moreover, the compounds or their salts can be used in a suitable hydrated form.

Regardless of the route of administration selected, a non-toxic but therapeutically effective quantity of one or more compounds disclosed herein is employed in the method herein. The dosage regimen for preventing or treating gastrointestinal motility disorders with the compounds is selected in accordance with a variety of factors, including disorder type, age, weight, sex, and medical condition of the patient, the severity of the gastrointestinal motility disorder, the route of administration, and the particular compound employed in the treatment. A physician or veterinarian of ordinary skill can readily determine and prescribe the effective amount of the compound required to prevent or arrest the progress of the condition. In so proceeding, the physician or veterinarian can employ relatively low doses at first and subsequently increase the dose until a maximum response is obtained.

The compounds described herein can be prepared by any available procedure. For example, some of the procedures are known in the art and, for the purposes of the practice of the method described herein, any suitable method of preparing the compounds is acceptable. The compounds useful in the method herein are generally prepared according to the reaction schemes set forth herein.

Some of the compounds herein are known compounds and methods of their syntheses are also known. Methods of syntheses are disclosed in the following list of U.S. Patents for some compounds useful in the method herein: 4,340,730; 3,901,876; 4,704,381; 3,881,056; 3,551,409; 4,593,019; 4,663,313; 4,614,732; 4,673,670; 4,683,223; 4,791,103; 4,714,697; 4,616,003; 4,626,526; 4,501,735; 4,755,594; 4,764,506; 4,600,707; 4,636,493; 4,738,954; and 4,582,823.

The following Reaction Scheme A shows the reaction sequence for preparing 6-N-substituted adenosine derivatives.

Scheme A

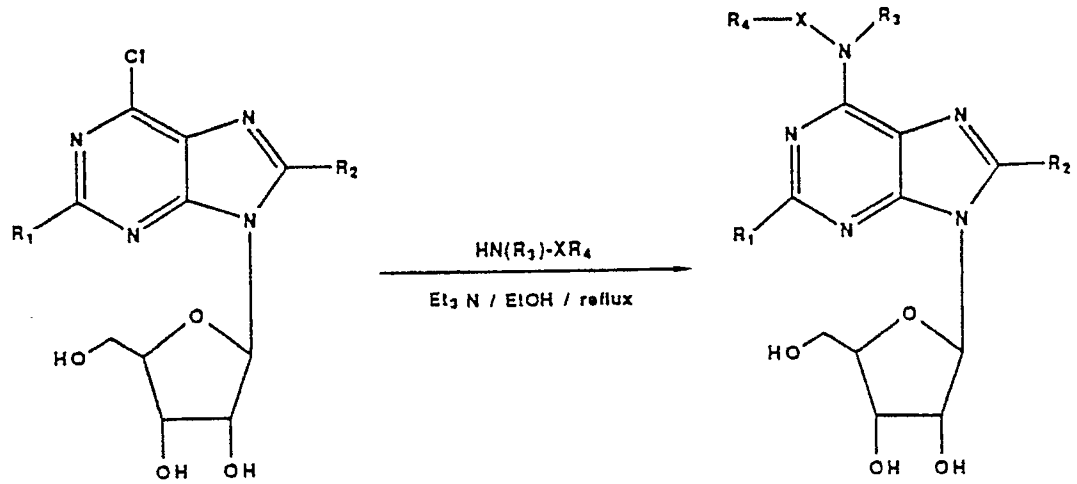


As illustrated in Scheme A, a 6-chloropurine riboside derivative is reacted with a suitable amine to produce the adenosine derivative product. The reaction is conducted in a mixture of triethylamine and ethanol and is heated to reflux to provide the adenosine derivative product.

The following examples are provided to illustrate the preparation of the adenosine derivative products useful herein using the reaction sequence shown in Scheme A. These examples, as well as all examples herein, are given by way of illustration only and are not to be construed as limiting the invention, either in spirit or scope, as many modifications, both in materials and methods, will be apparent from this disclosure to those skilled in the art. In these examples, temperatures are given in degrees Celsius (° C) and quantities of materials in grams and milliliters unless otherwise noted.

Example 1

N-[1-(phenylmethyl)-4-piperidinyl]adenosine

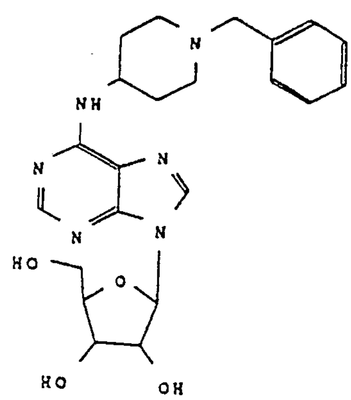

A suspension of 6 chloropurine riboside (1.5 g, 5.2 mmol) in absolute ethanol (20 ml) was formed. To this suspension was added 4-amino-1-benzyl piperidine (1.08 g, 5.7 mmol) (commercially available) plus triethylamine (0.72 g, 7.2 mmol). The reaction mixture was heated under reflux for 88 hours. The reaction mixture was cooled to room temperature and the solvent was removed in vacuo . The resulting residue was chromatographed on silica gel eluting with an eluant consisting of 12 parts ammonia saturated ethanol EtOH ($NH_3$) and 88 parts dichloromethane which yielded 1.87 g (82% yield) of the title product. Recrystallization from ether/methanol gave rosettes having a melting point of 176-182° C. MS: Calculated for $C_{22}H_{28}N_6O_4$,

441; found 441.

| Combustion analysis for $C_{22}H_{28}N_6O_4$: | | | |
|---|---|---|---|
| Calculated:<br>Found: | C, 59.99;<br>C, 59.70; | H, 6.41;<br>H, 6.46; and | N, 19.08.<br>N, 19.08. |

## Example 2

N-[2-(diethylamino)ethyl]adenosine

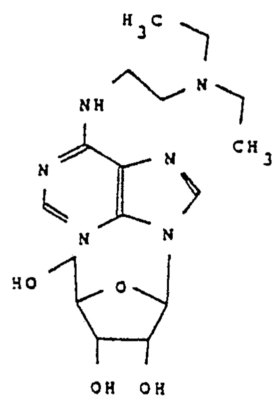

The experiment of Example 1 was repeated in every essential detail with the exception that 10 g (34.9 mmol) of 6-chloropurine riboside was suspended in 100 ml of absolute ethanol. To this suspension was added 5.07 g, (43.6 mmol) of N,N-diethylethylenediamine (commercially available) plus 5.29 g (52.3 mmol, 1.5 eq.) of triethylamine. Following reflux for 72 hours the residue was separated by chromatography on silica gel using a 10:90 ratio of MeOH($NH_3$) to dichloromethane. The title product was collected, yielding 3.03 g (48%). MS for $C_{16}H_{26}N_6O_4$: Calculated, 367; observed, 367.

| Combustion analysis for $C_{16}H_{26}N_6O_4$: | | | |
|---|---|---|---|
| Calculated:<br>Found: | C, 52.45;<br>C, 52.65; | H, 7.15;<br>H, 7.34; | N, 22.94.<br>N, 22.70. |

## Example 3

N-[3-(diethylamino)propyl]adenosine

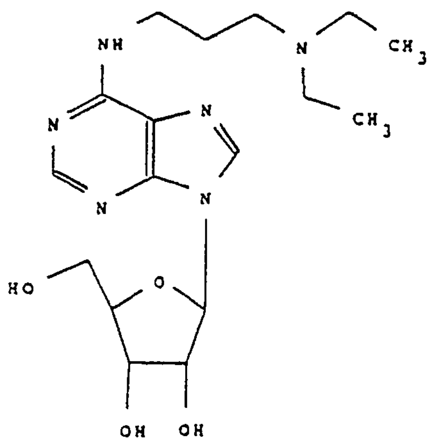

The experiment of Example 1 was repeated in every essential detail except that 2.0 g (7.0 mmol) of the 6-chloropurine riboside was suspended in 21 ml of absolute ethanol. To this suspension was added 1.0 g (7.7 mmol) of N,N-diethyl-1,3-propanediamine (commercially available) and 1.06 g (10.5 mmol, 1.5 eq) of triethylamine. The reaction mixture was refluxed for 68 hours and cooled to room temperature. The title product was separated by chromatography on silica gel using MeOH ($NH_3$) and dichloromethane in a ratio of 25:75. The title product was collected to yield 2.39 g (90%) of the product For $C_{17}H_{28}N_6O_4$ MH+ calculated: 381; observed: 381.

| Combustion analysis for $C_{17}H_{28}N_6O_4.3/2H_2O$: | | | |
|---|---|---|---|
| Calculated: | C, 50.11; | H, 7.67; | N, 20.62. |
| Found: | C, 50.16; | H, 7.24; | N, 20.34. |

Example 4

N-[2-(diethylamino)ethyl]-N-methyladenosine

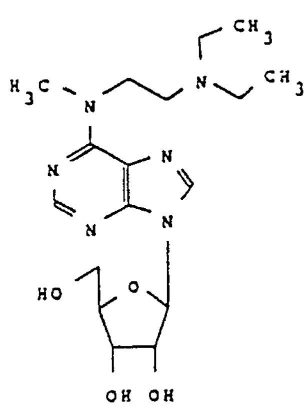

The experiment of Example 1 was repeated in every essential detail except for the following. The suspension of 6-chloropurine riboside was formed using 2.0 g (7.0 mmol) of the riboside in 14 ml of absolute ethanol. N,N-diethyl-N$^1$-methylethylenediamine (commercially available) was added to the suspension in an amount of 1.1 g (8.4 mmol) along with 1.06 g (10.5 mmol, 1.5 eq) of triethylamine. The reaction mixture was refluxed for 72 hours and subsequently cooled to room temperature to yield the product. The title product was separated by chromatography on a silica gel column eluting with an eluant that was 10 parts methanol, 90 parts dichloromethane and 0.5 parts ammonium hydroxide ($NH_4OH$). The title product was collected in an amount of 1.47 g (55%) and had a melting point of 63-66°. For $C_{17}H_{28}N_6O_4$ $M^+$ calculated: 380; observed: 380

| Combustion analysis: $C_{17}H_{28}N_6O_4.1/2H_2O$ | | | |
|---|---|---|---|
| Calculated: | C, 52.43; | H, 7.51; | N, 21.58. |
| Found: | C, 52.97; | H, 7.46; | N, 21.54. |

## Example 5

N-[2-(1H imidazol-4-yl)ethyl]adenosine, hydrochloride

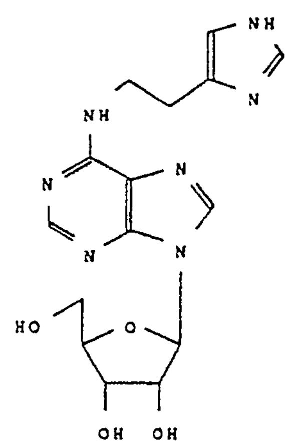

The experiment of Example 1 was repeated in every essential detail with the following exceptions. A suspension of 6-chloropurine riboside was formed by suspending 2.0 g (7.0 mmol) in 14 ml of absolute ethanol. To the suspension was added 1.41 g (7.7 mmol) of histamine dihydrochloride (commercially available).

Triethylamine was added to the suspension in an amount of 2.8 g (28 mmol) and the reaction mixture was refluxed for 96 hours. The solvent was removed in vacuo and the residue redissolved in 60 ml $H_2O$/MeOH (1:1). The resulting solution was treated with 3.09g (22.3 mmol) of potassium carbonate. The resulting suspension was stirred for 0.5 hours, filtered and the filtrate concentrated in vacuo giving a residue which was chromatographed on silica gel eluting with an eluant of 15 parts methanol, 85 parts dichloromethane and 1 part ammonium hydroxide to yield 1.37 g (52.3%) of the title product. The resultant product had a melting point of 133-145°. MS calculated: 362; observed, 362.

| Combustion analysis for $C_{15}H_{19}N_7O_4.1/4HCl.1/4H_2O$ | | | | |
|---|---|---|---|---|
| Calculated: | C, 48.05; | H, 5.31; | N, 26.15; | O, 2.36. |
| Found: | C, 48.17; | H, 5.54; | N, 25.37; | O, 2.29. |

## Example 6

N-[2-(1-pyrrolidinyl)ethyl]adenosine, monohydrochloride

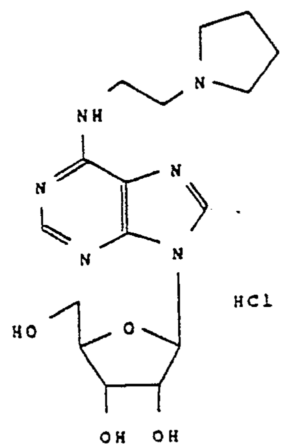

The experiment of Example 1 was repeated in every essential detail with the following exceptions. The suspension of 6-chloropurine riboside was formed using 1.80 g (6.3 mmol) in 12 ml of absolute ethanol. To this suspension was added 2-(1-pyrrolidinyl)ethylamine (commercially available) in an amount of 0.93 g, (8.2 mmol). Triethylamine 0.95 g (9.4 mmol, 1.5 eq) was added and the reaction mixture refluxed for 68 hours. Concentration in vacuo gave a residue which was chromatographically purified on silica gel eluting with an eluant of 10 parts methanol, 90 parts dichloromethane, and 0.5 parts ammonium hydroxide giving a glass which was crystallized from ethanol/acetone at -60° C to give the hydrochloride salt.

The title product was collected in an amount of 0.37g (16%) and had a melting point of 95-102° C. MS: Calculated, 365; observed, 365.

| Combustion analysis for $C_{16}H_{24}N_6O_4.HCl.1/2H_2O$ | | | | |
|---|---|---|---|---|
| Calculated:<br>Found: | C, 46.88;<br>C, 46.72; | H, 6.39;<br>H, 6.34; | N, 20.50;<br>N, 19.84; | Cl, 8.65.<br>Cl, 8.14. |

## Example 7

cis N-1-[3-(4-fluorophenoxy)propyl]-3-methoxy-4-piperidinyl]adenosine

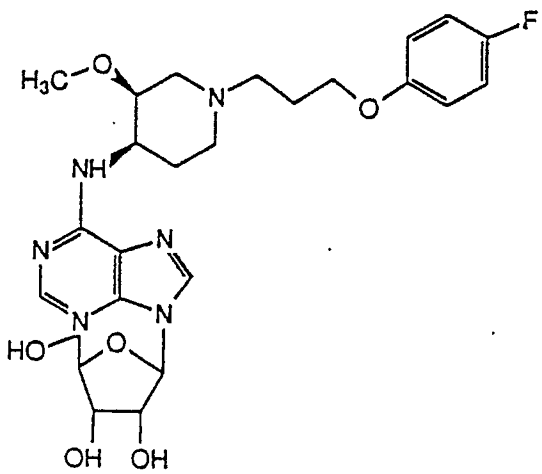

The experiment of Example 1 was repeated in every essential detail with the following exceptions. A suspension was formed using 1.25 g (4.43 mmol) of 6-chloropurine riboside in 15 ml of absolute ethanol. The amine (made in accordance with the teachings of European patent 76530) having the following structure was added in an amount of 1.25 g (4.43 mmol), along with 0.67 g (6.6 mmol) of triethylamine.

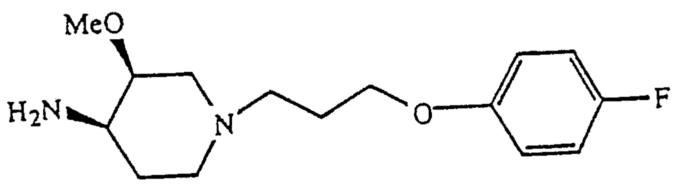

The reaction mixture was refluxed for 96 hours. The resulting residue was separated by chromatography on silica gel eluting with a mixture of 10 parts MeOH ($NH_3$) and 90 parts dichloromethane. The title product was yielded in an amount of 1.5 g (64%) and had a melting point of 77-87°. MS: Calculated, 533; observed, 533.

| Combustion analysis for $C_{25}H_{33}N_6O_6F$: | | | | |
|---|---|---|---|---|
| Calculated:<br>Found: | C, 56.38;<br>C, 56.36; | H, 6.25;<br>H, 6.34; | N, 15.78;<br>N, 15.40; | F, 3.57.<br>F, 3.40. |

Example 8

endo-N-(8-methyl-8 azabicyclo[3.2.1]octan-3-yl)adenosine, monohydrochloride

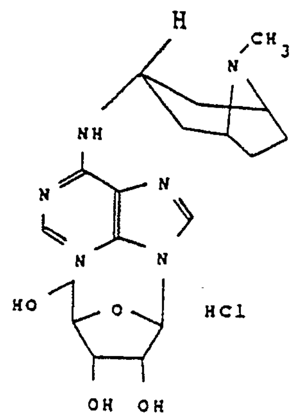

The experiment of Example 1 was repeated in every essential detail with the following exceptions. A suspension of 2.00 g (6.98 mmol) of 6-chloropurine riboside and 14 ml of absolute ethanol was formed. To the suspension was added 0.98 g (6.98 mmol) of an amine having the following structure [produced in accordance with the procedure in J. Am. Chem. Soc. 79, 4194 (1957)]

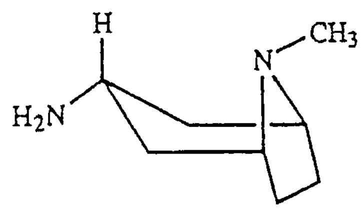

and 0.85 g (8.37 mmol) of triethylamine. The reaction mixture was refluxed for 96 hours. The reaction mixture after heating was a thick suspension which was cooled to room temperature and filtered, washed with absolute ethanol, and dried to yield 2.6 g (87%) of the named product having a melting point of 261-262°. MS: Calculated, 391; observed, 391.

| Combustion analysis for $C_{18}H_{26}N_6O_4$.HCl | | | | |
|---|---|---|---|---|
| Calculated:<br>Found: | C, 50.64;<br>C, 50.62; | H, 6.37;<br>H, 6.42; | N, 19.69;<br>N, 19.57; | Cl, 8.30.<br>Cl, 8.38. |

## Example 9

N-[2-(1-piperidinyl)ethyl]adenosine

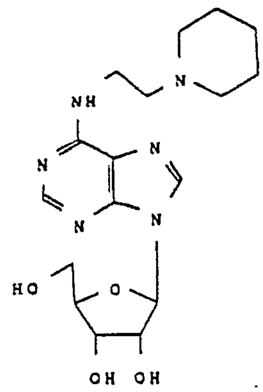

The experiment of Example 1 was repeated in every essential detail except for the following. The suspension was prepared using 2.0g (6.98 mmol) of the 6-chloropurine riboside in 30 ml of absolute ethanol. 1-(2-Aminoethyl) piperidine (commercially available) was added in an amount of 1.07 g (0.7 ml, 8.3 mmol). Also added to this suspension was 1.5 ml (10.5 mmol) of triethylamine. The reaction mixture was refluxed for 72 hours. The resulting residue was chromatographed on a silica gel column eluting with an eluant of 20 parts methanol, 79 parts dichloromethane and one part ammonium hydroxide. The title product was produced in a yield of 1.1 g (42%). MS: Calculated, 379; observed, 379.

| Combustion analysis yielded for $C_{17}H_{26}N_6O_4.0.4EtOH$ | | | |
|---|---|---|---|
| Calculated: | C, 53.87; | H, 7.21; | N, 21.18. |
| Found: | C, 53.68; | H, 7.22; | N, 21.15. |

Example 10

N-[2-(dipropylamino)ethyl]adenosine

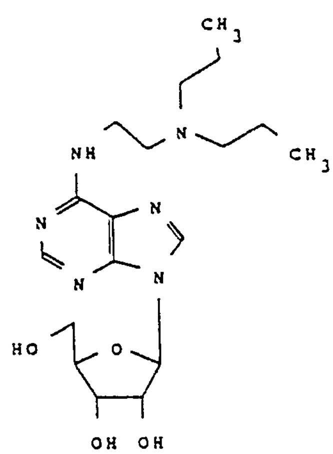

The experiment of Example 1 was repeated in very essential detail with the following exceptions. A suspension of 2.0g (6.98 mmol) of 6-chloropurine riboside in 30 ml of absolute ethanol was formed. To the

suspension was added 1.17 g (8.37 mmol) of N,N-di-n-propylethylene diamine (commercially available) and 1.5ml (10.5 mmol) of triethylamine. The reaction mixture was refluxed for 72 hours at which time the residue was separated by chromatography on a silica gel column using an eluant of 20 parts methanol, 79 parts dichloromethane and 1 part ammonium hydroxide to yield 2.15 g (78%) of the title compound DSC = 130.7 - 134.4°. MS: Calculated, 395; observed, 395.

| Combustion analysis for $C_{18}H_{30}N_6O_4$: | | | |
|---|---|---|---|
| Calculated: | C, 54.81; | H; 7.66; | N, 21.30. |
| Found: | C, 55.19; | H, 7.80; | N, 21.03. |

## Example 11

exo-N-(8-methyl-8-azabicyclo[3.2.1]octan-3-yl)adenosine, monohydrochloride

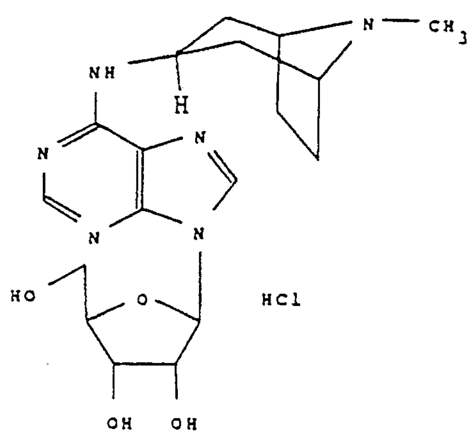

The experiment of Example 1 was repeated in every essential detail with the exception of the following. A suspension was formed containing 2.0 g of 6-chloropurine riboside (6.98 mmol) in 30 ml of absolute ethanol. To the suspension was added 1.17 g (8.37 mmol) of an amine having the following structure (formed in accordance with the procedure described in Berichte 31, 1202 (1898))

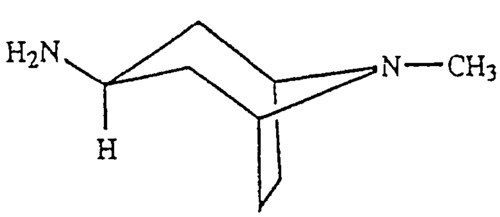

and 1.5 ml (10.5 mmol) of triethylamine. The reaction mixture was refluxed for 90 hours. The reaction mixture after reflux was a thick suspension which was cooled to room temperature and filtered, washed with absolute ethanol, and dried to give 2.2 g of the desired product (74% yield), m.p. 153 - 171 °C

| Combustion analysis for $C_{18}H_{26}N_6O_4$.HCl | | | | |
|---|---|---|---|---|
| Calculated:<br>Found: | C, 50.64;<br>C, 49.82; | H; 6.37;<br>H, 6.47; | N, 19.69;<br>N, 19.16; | Cl, 8.30.<br>Cl, 8.12. |

## Example 12

N,N-diethyl-N-methyl-2-[(9β-D-ribofuranosyl-9H-purin-6-yl)amino]ethaneammonium iodide

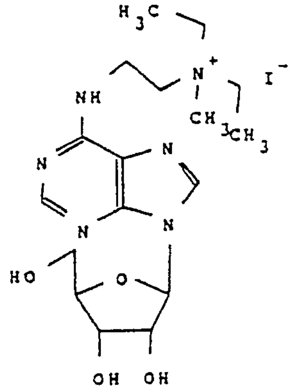

To a solution of 0.70 g (1.9 mmol) of the title compound of Example 2 in 6 ml $CH_3CN$/MeOH (2:1) was added 0.30 g (2.1 mmol) of methyl iodide. The reaction mixture was stirred for 15 hours. An additional quantity of methyl iodide (0.054 g, 0.38 mmol) was added and the resulting solution stirred for an additional 1.5 hours. The solution was concentrated in vacuo yielding 0.920 g of the title compound (95% yield) as a colorless foam (mp 86 - 102° C).

| Combustion analysis for $C_{17}H_{29}N_6O_4I$ | | | | |
|---|---|---|---|---|
| Calculated:<br>Found: | C, 40.17;<br>C, 40.02; | H; 5.75;<br>H, 5.85; | N, 16.53;<br>N, 16.60; | I, 24.96<br>I, 24.76. |

## Example 13

N-[2-(2,5-dihydro-1H-pyrrol-1-yl)ethyl]adenosine, monohydrochloride

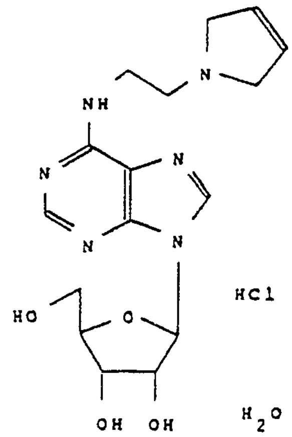

To a suspension of 6 chloropurine riboside (1.27 g, 4.42 mmol) in 1 ml of absolute ethanol was added 2-(2,5-dihydro-1H-pyrrol-1-yl)-1-ethanamine (prepared in accordance with Arzneim. Forsch. 21 (12) 2089 (1971)) in an amount of 0.54 g (4.9 mmol). The reaction mixture was heated under reflux for 40 hours. Concentration of the reaction mixture in vacuo gave a residue which was chromatographed on cellulose powder (Whatman CC 31). The eluant was 85 parts n-butanol and 15 parts water. Fraction monitoring was accomplished by thin layer chromatography on silica gel (Kieselgel 60 F254) eluting with a mixture of 20 parts methanol, 79 parts methylene chloride and one part ammonium hydroxide. The chromatography gave a glass which was azeotroped with water 3 times to remove the n-butanol. Lyophilization of the aqueous solution gave the title compound in an amount of 0.41 g (21% yield) as a white powder (mp 88-105 °C) MS MH+ Calculated: 363, found 363.

| Combustion analysis for $C_{16}H_{22}N_6O_4.HCl.H_2O$ | | | | |
|---|---|---|---|---|
| Calculated: | C, 46.10; | H, 6.05; | N, 20.16; | Cl, 8.50. |
| Found: | C, 46.35; | H, 5.82; | N, 20.28; | Cl, 8.95. |

## Example 14

N-[2-(1H-imidazol-1-yl)ethyl]adenosine, hydrochloride

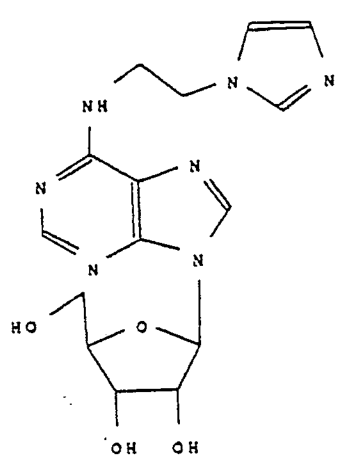

To a suspension of 6-chloropurine riboside (1.63 g, 5.69 mmol) in 12 ml of absolute ethanol was added 0.63 g (5.7 mmol) of 2-(imidazol-1-yl)ethylamine (prepared in accordance with Z.Obsch. Chem. 9 1933 (1939); Chem Ab. 1940 2466) plus 0.80 g (8.0 mmol) of triethylamine. The reaction mixture was heated under reflux for 77 hours. Removal of the solvent in vacuo and chromatography of the residue on silica gel eluting with a mixture of ammonia saturated methanol, MeOH($NH_3$), and dichloromethane in a ratio of 14:86 gave the free base of the title compound in an amount of 1.2 g (59% yield). The material was converted to the hydrochloride salt by suspending the material in 20 ml of water at 0° centigrade and adding 1N hydrochloric acid until a pH of 2.6 was attained. The solution was frozen and lyophilized to a white powder which was passed through a column of cellulose powder eluting with a mixture of 85 parts n-butanol and 15 parts water. The appropriate fractions were combined and concentrated to give a glass which was azeotroped twice with water and then dissolved in water, frozen and lyophilized yielding 0.97 g of the title compound (41% yield) as a white powder. MS: MH+ Calculated: 362 Found: 362

| Combustion analysis for $C_{15}H_{19}N_7O_4.1.4HCl.0.7H_2O$ | | | | |
|---|---|---|---|---|
| Calculated:<br>Found: | C, 42.39;<br>C, 42.34; | H, 5.17;<br>H, 5.05; | N, 23.07;<br>N, 23.58; | Cl, 11.68.<br>Cl, 11.81. |

## Example 15

N-[2-(2-methyl-1H-imidazol-1-yl) ethyl]adenosine, hydrochloride

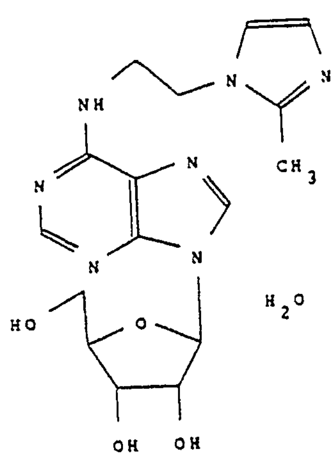

A suspension was prepared containing 1.64 g (5.74 mmol) of 6-chloropurine riboside in 12 ml of absolute ethanol. To the solution was added 0.72 g (5.7 mmol) of 2-(2-methyl-imidazol-1-yl) ethylamine (prepared in accordance with Z.Obsch. Chim 11 , 545 (1941); Chem Ab. 1941 6938) and 0.81 g (8.0 mmol) of triethylamine. The reaction mixture was heated under reflux for 77 hours. The solvent was removed in vacuo and chromatography of the residue on silica gel was performed by eluting with a mixture of 22 parts methanol, 88 parts methylene chloride, and one part ammonium hydroxide. The title compound was obtained as the free base yielding 1.2 g (55% yield). This material was converted to the hydrochloride salt by suspending in 20 ml of water at 0° and adding 1 normal hydrochloric acid until a pH of 2.6 was attained. The solution was frozen and lyophilized giving a white powder which was passed through a column of cellulose powder eluting with a mixture of 85 parts n-butanol and 15 parts water. The appropriate fractions were combined and concentrated to give a glass which was azeotroped and then dissolved in water, frozen and lyophilized giving 1.1 g (44% yield) of the title compound as a white powder. MS: MH+ Calculated:

376 Found: 376

| Combustion analysis for $C_{16}H_{21}N_7O_4.1.1HCl.H_2O$ | | | | |
|---|---|---|---|---|
| Calculated: | C, 44.33; | H, 5.60; | N, 22.62; | Cl, 8.45 |
| Found: | C44.18; | H, 5.27; | N, 22.79; | Cl: 9.21. |

## Examples 16-18

The three adenosine compounds comprising these three examples are commercially available compounds. Their structures are as follows:

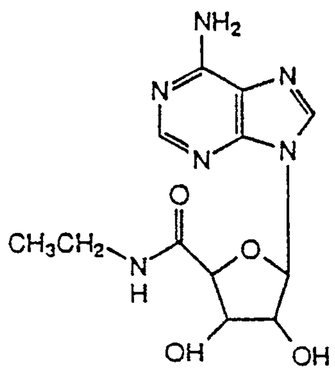

Example 16

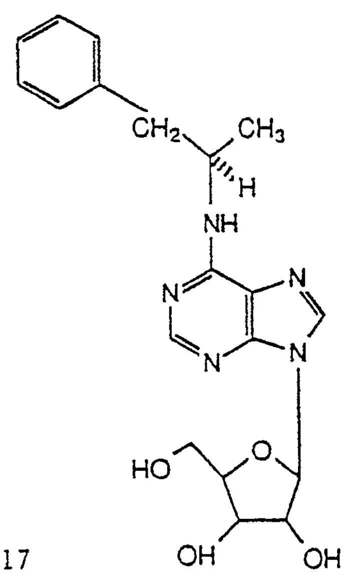

Example 17

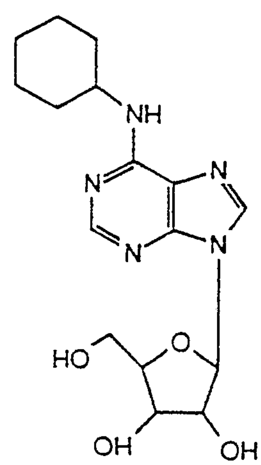

Example 18

The compounds herein have been shown to be useful for treating gastrointestinal motility disorders in mammals. Their usefulness has been shown by the demonstrated prokinetic activity of representative compounds. Prokinetic activity of any compound can be determined by measuring the enhancement of gastric emptying of a meal in a rat model to which the compound has teen administered. This method for determining the prokinetic activity of a compound has been described by Droppleman, et al, J. Pharmacol. and Methods 4 : 227-230 (1980).

Rat Gastric Emptying Protocol

A test meal for measuring gastric emptying in rats was prepared. Ten grams of methylcellulose (2% solution = 15 centipoises, Aldrich Chemical Company, Milwaukee, WI) was added to 200 ml of cold water and mixed at 20,000 rpm in a Waring blender to insure dispersion and hydration of the methylcellulose. In addition, two beef bouillon cubes (Wyler's, Columbus, OH) dissolved in 100 ml of warm water were added to the mixture, followed by 16 g of casein (Hammersten, Schwartz/Mann, Orangeburg, NY), 8 g of powdered confectioners sugar and 8 g of cornstarch. The ingredients were mixed for two minutes at 20,000 rpm and the resultant test meal was refrigerated for 48 hours to allow trapped air to escape. Male Charles River Rats, Crl: COBS, CD (SD) BR Strain, 180-200 g body weight, were used in groups of six animals. The animals were food deprived for 24 hours prior to the experiment with access to water ad libitum . The compounds to be evaluated were prepared in a 0.5% aqueous methylcellulose solution. If insoluble, the mixture was homogenized for two minutes at 5500 rpm using a Try-R-Stir-R. The compounds were injected intraperitoneally at a volume of 5 ml/kg, 30 minutes before the test meal, (3.0 ml/rat i.g.). Control animals received only the vehicle. Sixty minutes after the test meal, the rats were sacrificed by cervical dislocation. The stomachs were removed intact and weighed. The stomachs were kept opened, gently rinsed with tap water, blotted dry with paper towelling, and the empty stomach weighed. The difference between the weight of the full and empty stomach is indicative of the amount of meal remaining in the stomach. The amount of meal remaining in the stomach was subtracted from the weight of 3 ml of the test meal to determine the amount of food emptied from the stomach during the test. Weight of the test meal was determined by weighing three samples (3 ml) at the beginning and three samples at the end of each experiment and calculating the mean. The mean and standard error of the amount of meal emptied were calculated. A dose of compound was considered active if emptying in 4 of 6 animals given the compound exceeded the median amount emptied for the control animals. These compounds were then tested for antral motor effects in conscious dogs.

Antral Motility in Conscious Fasted Dogs

Gastric antral contractile activity is stimulated by prokinetic drugs which enhance gastric emptying of solid food as has been shown by Jacoby et al, Gastroenterology, 52 676-684 (1967). This contractile activity is thought to enhance gastric emptying by more rapidly reducing food particle size for passage through the pylorus. The ability of a test compound to increase the frequency and/or amplitude of the contractile activity is a measure of gastrointestinal prokinetic activity of the compound.

Mongrel dogs of either sex were surgically implanted with strain gauge force transducers on the gastric antrum at 6 cm, 4 cm and 2 cm from the gastroduodenal junction. The dogs were allowed at least two weeks to recover and were trained to stand quietly in Pavlov slings.

Dogs were fasted for 18 to 24 hours prior to each experiment to record a pattern of antral contractile activity characteristic of the fasted state called the Migrating Motor Complex (MMC). The period of the MMC cycle is approximately 90 to 120 minutes and consists of 45 to 60 minutes of motor quiescence (Phase I) 30 to 45 minutes of intermittent activity (Phase II) and 10 to 15 minutes of intense contractile activity (Phase III). A controlled MMC period is recorded prior to compound administration to obtain the length of the quiescent Phase I period. Compound is given intravenously at the end of Phase III of the control MMC cycle and a subsequent Phase I period is examined for the ability of the compound to produce contractions of a determined duration.

Table I provides the results of the rat gastric emptying evaluation and the dog fasted antral motility evaluation of representative compounds herein. In the table, the indicated result for the rat gastric emptying is the percentage increase in gastric emptying at a dose of 10 milligrams per kilogram (mpk) administered intraperitoneally (IP). The metoclopramide value at 10.0 mpk (IP) is the value given in parentheses in the Table. The results for the dog fasted antral motility study are reported as the dose in milligrams per kilogram (mpk) administered intravenously and the duration in minutes of antral motility. The data in Table I reported for the rat gastric emptying studies for Examples 13, 15, 17 and 18 were conducted at a dose of 3 milligrams per kilogram. The data in Table I reported for the rat gastric emptying study for Example 16 was conducted at a dose of 1 milligram per kilogram.

TABLE I

| | | Dog Fasted Antral Motility | |
|---|---|---|---|
| Example No. of Compound | Rat Gastric Emptying | Dose (mpk) | Duration |
| 1 | -7.1 (----) | --- | --- |
| 2 | 18.3 (27.6) | 3.0 | 48 min. |
| | | 10.0 | 60 min. |
| 3 | 16.7 (32.9) | 3.0 | 60 min. |
| | | 10.0 | 60 min. |
| 4 | -4.1 (33.3) | 3.0 | 30 min. |
| 5 | 29.5 (14.2) | 0.3 | 40 min. |
| | | 3.0 | 52 min. |
| 6 | 24.3 (30.8) | --- | --- |
| 7 | 2.0 (30.0) | --- | --- |
| 8 | 16.7 (43.1) | 3.0 | 49 min. |
| | | 10.0 | 60 min. |
| 9 | 14.3 (30.0) | --- | --- |
| 10 | 0.8 (24.0) | --- | --- |
| 11 | 15.1 (----) | 3.0 | 52 min. |
| | | 10.0 | 60 min. |
| 12 | 4.7 (27.5) | --- | --- |
| 13 | 15.5 (17.0) | 3.0 | 60 min. |
| 14 | 20.2 (14.0) | 0.3 | 35 min. |
| | | 3.0 | 60 min. |
| 15 | 6.7 (14.0) | 3.0 | 60 min. |
| 16 | 15.7 (25.8) | --- | --- |
| 17 | 34.6 (----) | --- | --- |
| 18 | 27.8 (----) | --- | --- |

## Claims

1. Use of a compound of the formula

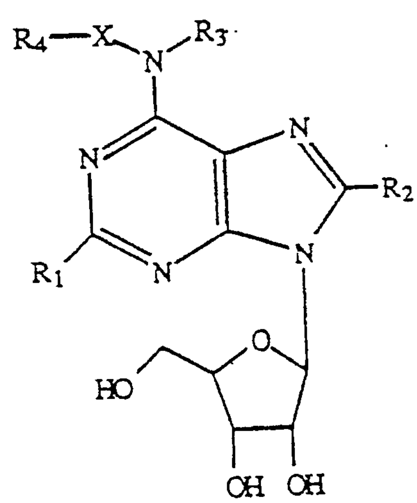

wherein each $R_1$ and $R_2$ is independently hydrogen, hydroxyl, halogen, alkyl, phenyl, alkoxy, morpholino, piperidino, piperazino, phenoxy, thiophenoxy or amino optionally substituted by alkyl, aralkyl, or phenyl;
wherein each $R_3$ can independently be hydrogen or lower alkyl;
wherein X can be a straight chain, branched chain or cyclic alkylene from 1 to 10 carbon atoms; and wherein $R_4$ can be optionally substituted imidazol-1-yl, imidazol-2-yl, imidazol-4-yl, pyrrolinyl, pyrrolidinyl, piperidinyl, triazolyl, hydroxyalkyl, dihydroxyalkyl, alkoxy alkyl, and dialkoxyalkyl,

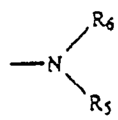

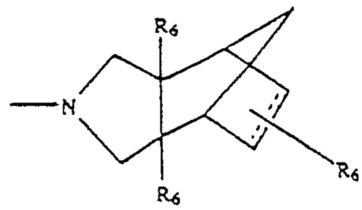

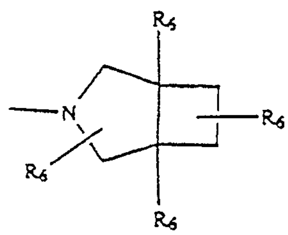

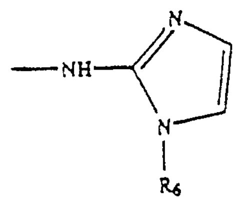

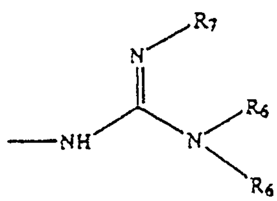

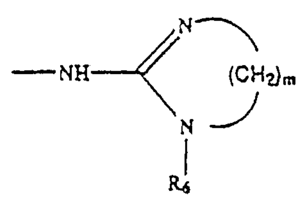

wherein X can be a valence bond and $R_4$ can be

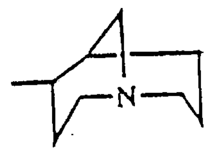

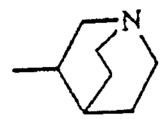

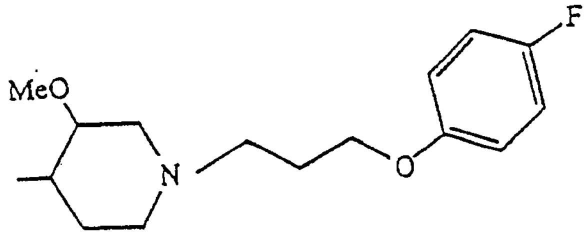

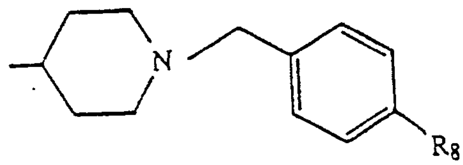

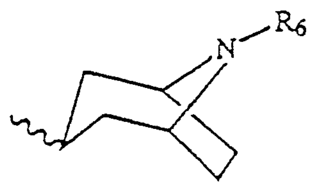

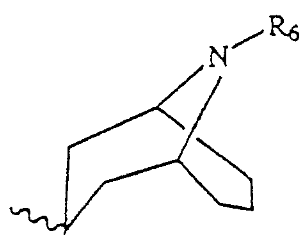

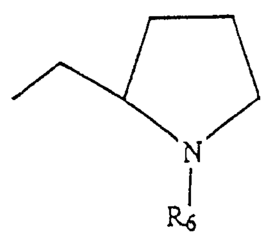

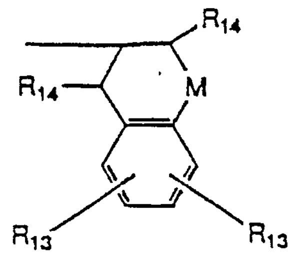
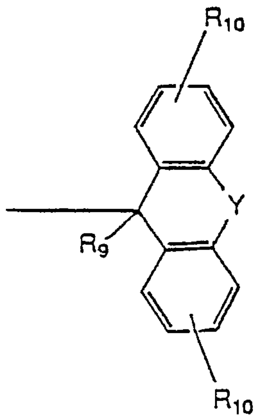
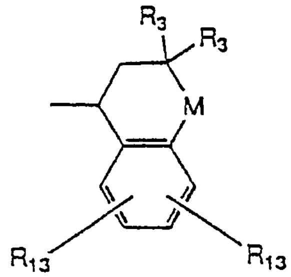
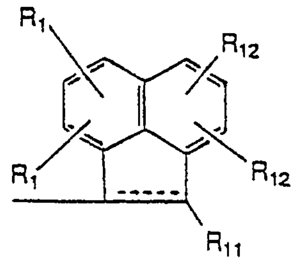
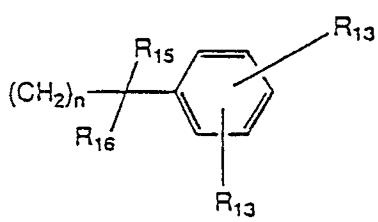
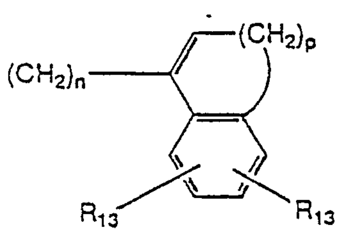
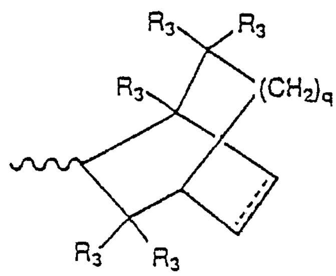
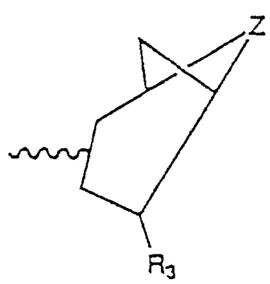
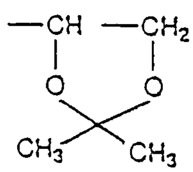
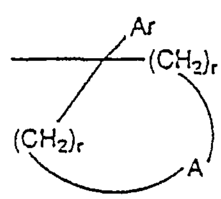
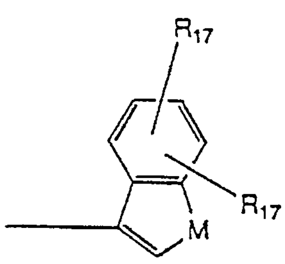
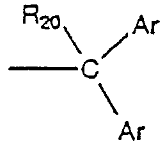
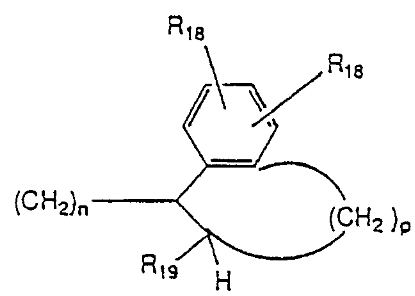

wherein each $R_5$ and $R_6$ can independently be hydrogen, alkyl, aralkyl, phenyl, and optionally substituted phenyl and aralkyl; and

wherein each $R_7$ can independently be hydrogen, alkyl, aralkyl, aryl, cyano or nitro;

wherein each $R_8$ can independently be hydrogen, alkyl, aralkyl, aryl or acyl;

wherein each $R_9$ can independently be hydrogen, hydroxy, lower alkyl, lower alkanoyloxy and benzoyloxy;

wherein each $R_{10}$ can independently be hydrogen, halogen, hydroxy, lower alkoxy, lower alkanoyloxy, benzoyloxy, lower $S(O)_q$ - alkyl, sulfonamide, trifluoromethyl, lower alkyl, amino, lower mono- or dialkylamino, nitro or sulfhydryl;

wherein each $R_{11}$ can independently be hydrogen, lower alkyl, hydroxy or lower alkoxy;
wherein each $R_{12}$ can independently be hydrogen, hydroxy, halogen, amino, nitro, lower alkyl, lower alkoxy or trifluoromethyl;
wherein each $R_{13}$ can independently be hydrogen, lower alkyl, lower alkoxy, hydroxy, lower alkanoyl, nitro, amino, trifluormethyl, halogen or taken together a methylenedioxy group;
wherein each $R_{14}$ can independently be hydrogen, hydroxy, or lower alkyl;
wherein each $R_{15}$ can independently be a straight or branched lower alkyl;
wherein each $R_{16}$ can independently be hydrogen, lower alkyl, lower alkoxy, or lower alkanoyloxy;
wherein each $R_{17}$ can independently be hydrogen, hydroxy, lower alkoxy, lower alkyl, amino, monoloweralkyl amino, diloweralkyl amino, nitro or halogen;
wherein each $R_{18}$ can independently be hydrogen, lower alkyl, lower alkoxy, hydrox , lower alkanoyl, nitro, trifluoromethyl, halogen, amino, monoloweralkyl amino, diloweralkyl amino, or taken together are a methylenedioxy group;
wherein each $R_{19}$ can independently be hydrogen, lower alkyl or lower alkanoyl;
wherein each $R_{20}$ can independently be hydrogen, hydroxy, lower alkyl, lower carboalkoxy, or lower alkanoyloxy;
wherein Y can be a valence bond, O, $S(O)_q$, NR in which R is H, lower alkyl, lower alkanoyl or benzoyl, $(CH_2)_s$, or -CH=CH-
wherein M can be O, S, SO or $SO_2$;
wherein Z can be $-C(CH_3)_2-$, $-CH_2-$, $-CH_2-CH_2-$ or -CH=CH-
wherein A can be a valence bond, O, S,

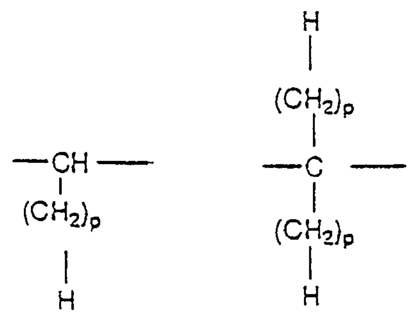

.

wherein each Ar can independently be (1) phenyl, (2) 1- or 2-naphthalenyl, (3)2-or 3-thienyl, (4)2-or 3-furanyl, (5)2-4-, or 5-thiazyl (6) 2-, 3-, or 4-pyridyl, or (7)2-pyrimidyl wherein each of (1), (2), (3), (4), (5), (6) or (7) is unsubstituted or substituted with at least one of lower alkyl, halogen, trifluoromethyl, hydroxy, lower alkoxy, lower acyloxy, amino. N-lower monoalkyl or N,N-lower dialkylamino, lower thioalkyl, lower alkylsulfonyl, or nitro;
wherein m can be 2 or 3;
wherein n can be an integer from 0 to 4;
wherein p can be an integer from 1 to 4;
wherein q can be an integer from 0 to 2;
wherein r can be an integer from 0 to 3;
wherein s can be 1 or 2; or
a pharmaceutically acceptable salt thereof, for preparing a medicament for treating gastrointestinal motility disorders in a mammal.

2. Use according to claim 1 wherein X is a valence bond and $R_4$ is

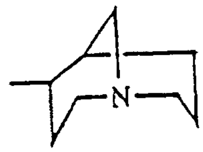

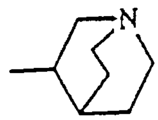

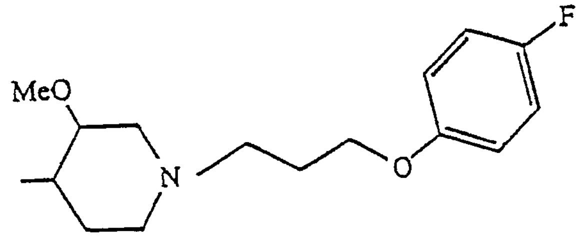

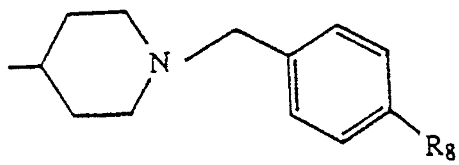

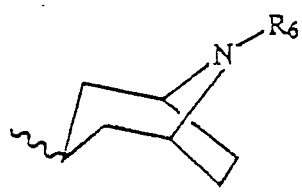

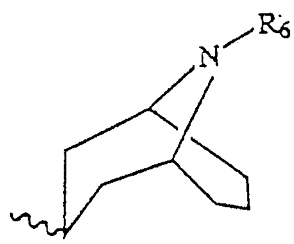

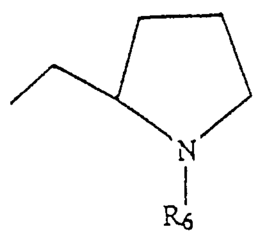

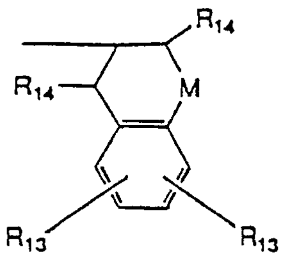

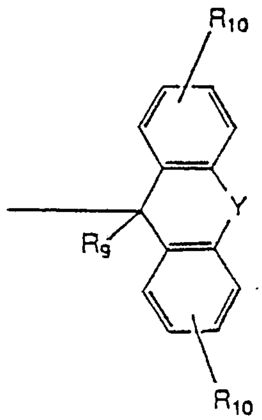

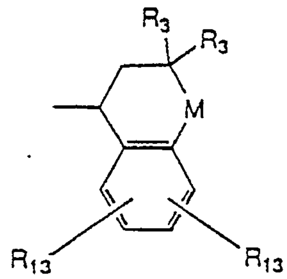

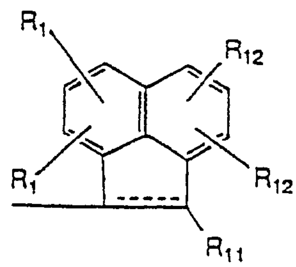

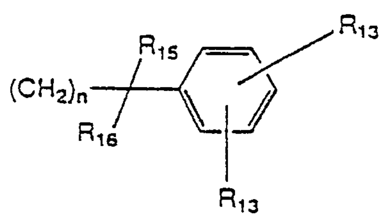

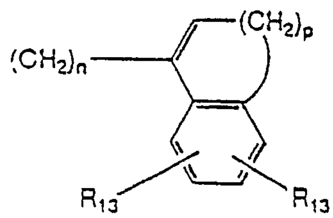

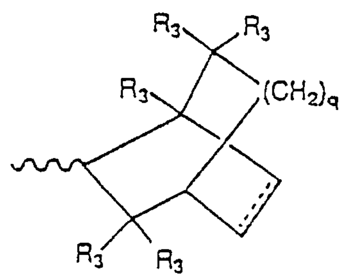

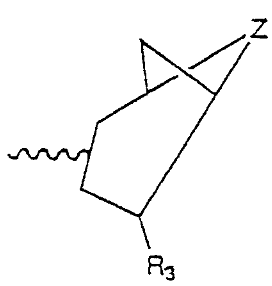

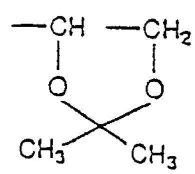

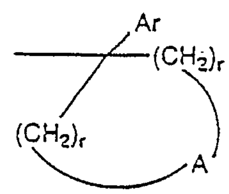

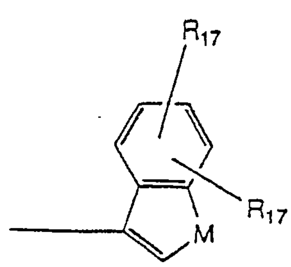

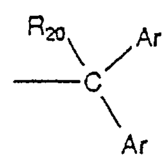

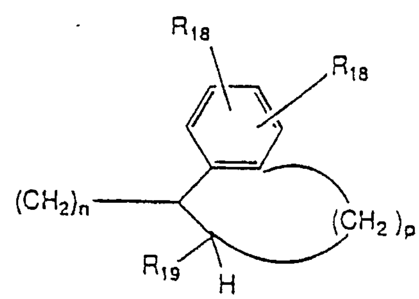

3. Use according to claim 2 wherein $R_4$ is

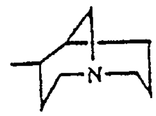

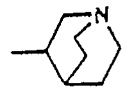

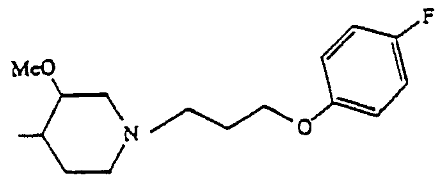

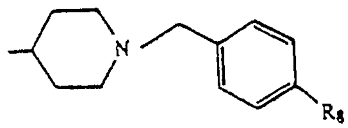

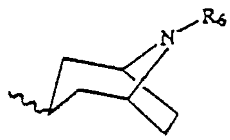

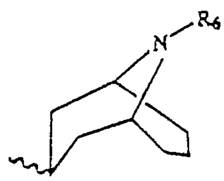

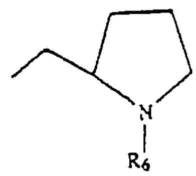

4. Use according to claim 1 wherein X is a straight chain, branched chain or cyclic alkylene from 1 to 10 carbon atoms and $R_4$ is imidazol-1-yl, imidazol-2-yl, imidazol-4-yl, pyrrolinyl, pyrrolidinyl, piperidinyl, triazolyl, hydroxyalkyl, dihydroxyalkyl, alkoxy alkyl or dialkoxyalkyl,

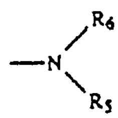

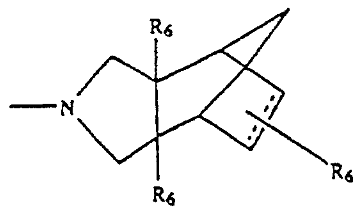

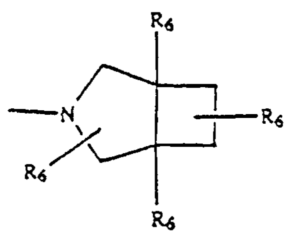

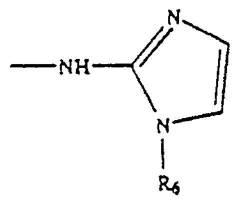

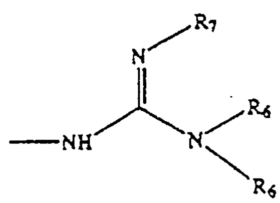

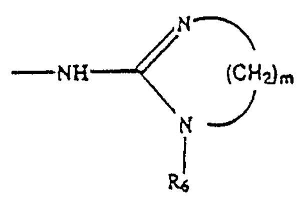

5. Use according to claim 1 wherein the compound is

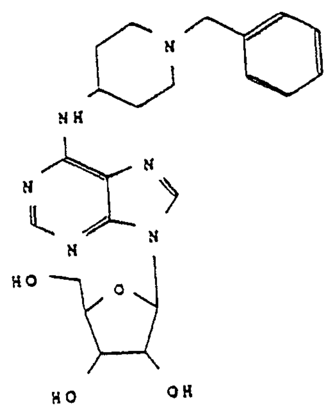

6. Use according to claim 1 wherein the compound is

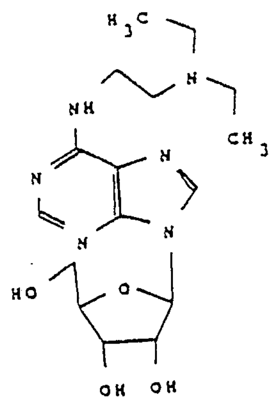

7. Use according to claim 1 wherein the compound is

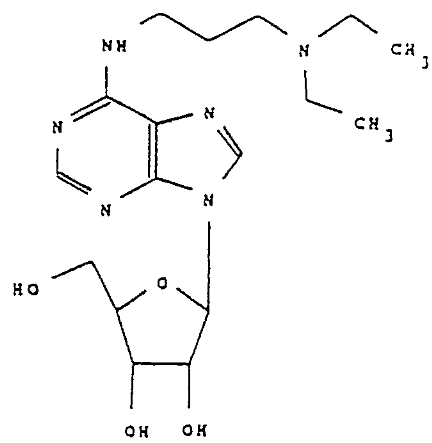

8. Use according to claim 1 wherein the compound is

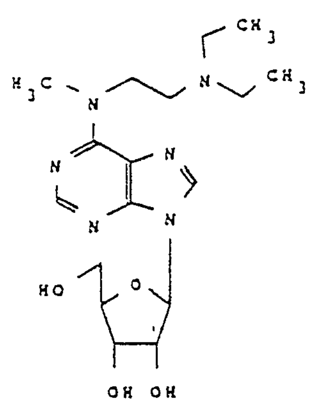

9. Use according to claim 1 wherein the compound is

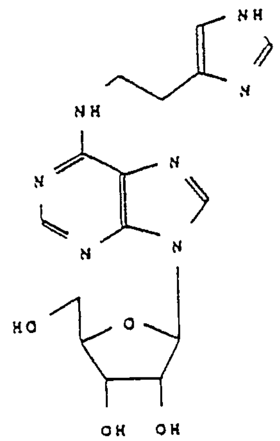

10. Use according to claim 1 wherein the compound is

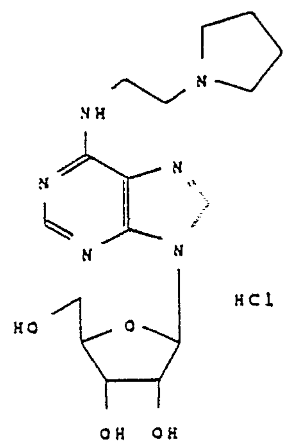

11. Use according to claim 1 wherein the compound is

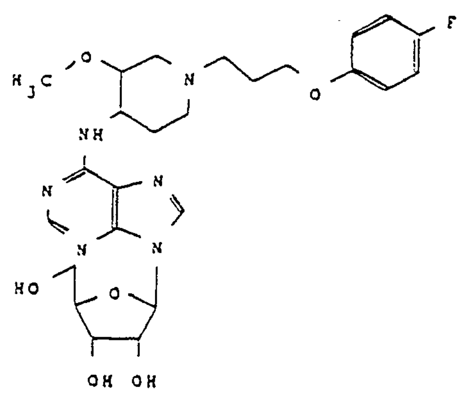

12. Use according to claim 1 wherein the compound is

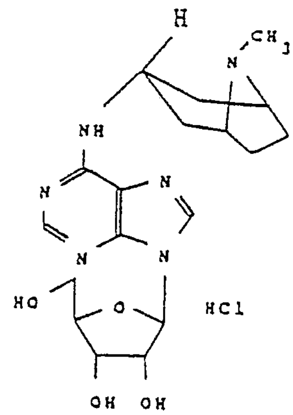

13. Use according to claim 1 wherein the compound is

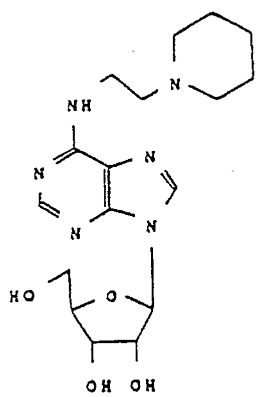

14. Use according to claim 1 wherein the compound is

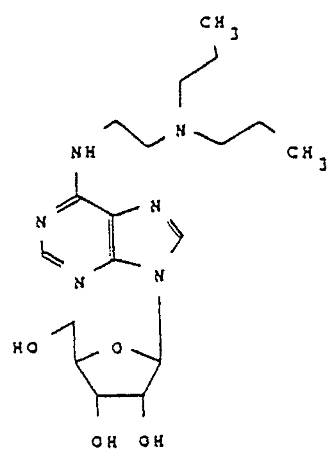

15. Use according to claim 1 wherein the compound is

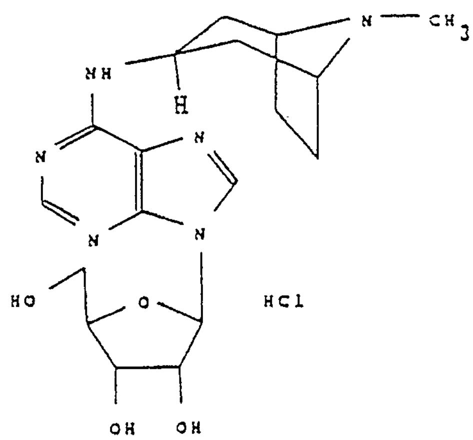

16. Use according to claim 1 wherein the compound is

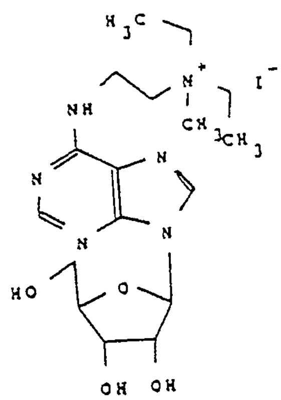

17. Use according to claim 1 wherein the compound is

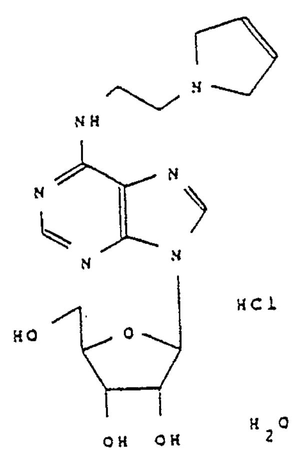

18. Use according to claim 1 wherein the compound is

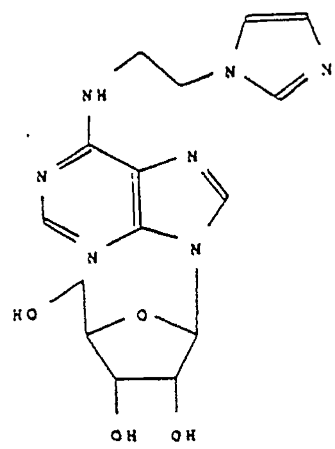

19. Use according to claim 1 wherein the compound is

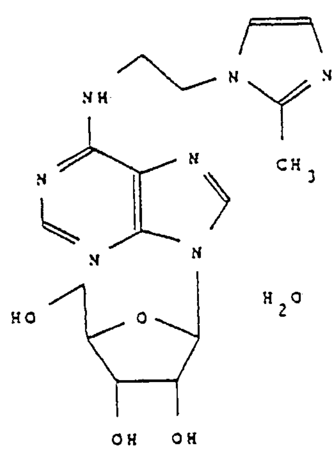

20. Use according to claim 1 wherein the compound is

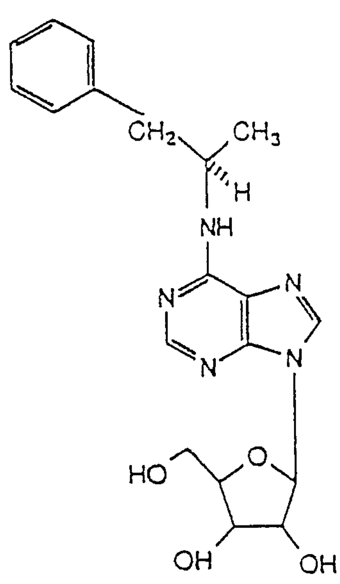

21. Use according to claim 1 wherein the compound is